# EUROPEAN PATENT APPLICATION

(11) **EP 4 803 616 A1**
(43) Date of publication of application: **09.09.2026**
(21) Application number: 24885686.6
(22) Date of filing: 29.10.2024
(51) Int. Cl.: C12N 5/10, A61K 35/17, A61P 35/00, C12N 5/0783, C12N 15/62, C12M 1/00, C12M 3/00

(54) **METHOD FOR PRODUCING CYTOTOXIC T CELLS**

(30) Priority: 30.10.2023 JP 2023185541
(71) Applicant: Takeda Pharmaceutical Company Limited, Osaka-shi, Osaka 541-0045 (JP)
(72) Inventor: HARA, Keisuke, Fujisawa-shi, Kanagawa 251-0012 (JP); HAYASHI, Tetsuji, Fujisawa-shi, Kanagawa 251-0012 (JP)
(74) Representative: Henderson, Helen Lee
(86) International application number: PCT/JP2024/038436
(87) International publication number: WO 2025/094915

(57) **Abstract**

Disclosed are a method for producing activated cytotoxic T cells derived from iPS cells, the method including the following step (1); (1) activating unactivated cytotoxic T cells derived from iPS cells in a container excellent in gas exchange, a method for proliferating activated cytotoxic T cells derived from iPS cells, the method including the following step (1A); (1A) activating unactivated cytotoxic T cells derived from iPS cells in a container excellent in gas exchange, a cell population containing activated cytotoxic T cells obtained by the method, and a medicine containing the cell population containing activated cytotoxic T cells.

## Description

### Technical Field

The present invention relates to a method for producing cytotoxic T cells, a method for proliferating cytotoxic T cells, a cell population containing cytotoxic T cells obtained by the method, a medicine containing the cell population containing cytotoxic T cells, and the like.

### Background of Invention

As one of cancer therapies, a CAR-T cell therapy using T cells (sometimes referred to as "CAR-T cells" in the present specification) expressing a chimeric antigen receptor (also referred to as "CAR" in the present specification) is known. CAR-T cells are, for example, produced using autologous T cells collected from patients themselves; however, high manufacturing costs and the risk of manufacturing failure are problems in running business. In particular, since as compared to healthy people, many cancer patients who receive CAR-T cell therapy have damaged T cells due to treatment with an anticancer agent and the like, the production efficiency of CAR-T cells from patient-derived T cells tends to be reduced.

One approach to solve such problems includes the development of allogeneic T cell sources. The technique of producing T cells from pluripotent stem cells may provide a platform for producing allogeneic homologous T cells. As such a technique, in NPL 1, it is reported that the differentiation efficiency of T cells is improved using induced pluripotent stem cells derived from an antigen-specific cytotoxic T cell clone (sometimes referred to as "iPS cells" in the present specification) or T cell receptor (referred to as "TCR" in the present specification)-transduced iPS cells as a starting material.

In NPL 2, it is reported that iPS cells (TCR-iPSC) into which an exogenous TCR gene has been transduced were produced, and CD8αβ cytotoxic T cells were regenerated from the iPS cells, and that the regenerated CD8αβ cytotoxic T cells showed cytotoxic activity equivalent to that of CD8αβ cytotoxic T cells regenerated from T cell-derived iPS cells.

When CAR-T cells are produced, a T cell activation step using a stimulatory substance (for example, an anti-CD3 antibody and an anti-CD28 antibody) is generally performed. In autologous CAR-T cell production, a container excellent in gas exchange in an activation step has been conventionally used. For example, in NPL 3 and NPL 4, it is described that a gas-permeable culture bag is used and gas exchange is performed in expansion culture of a process for producing autologous CAR-T cells. In NPL 5 and NPL 6, it is described that a gas-permeable culture bag is used in an activation step of autologous T cells.

On the other hand, an activation step of iPS cell-derived cytotoxic T cells is conventionally performed using a T flask or a well plate. Therefore, there is no report on the use of a container such as a gas-permeable culture bag as described above in the activation step of iPS-derived cytotoxic T cells.

### Citation List

### Non-patent Literature

NPL 1: Nature Communications, 2021, 12, 430
NPL 2: Molecular Therapy Methods & Clinical Development, 2020, 19, 250-260
NPL 3: Cytotherapy, 2019, 21, 327-340
NPL 4: J. Immunother, 2009, 32(2), 169-180
NPL 5: Dynabeads CD3/CD28 Catalog no. 40203D package insert
NPL 6: Takara Bio Inc. homepage "T lymphocyte (T cell) expansion culture by retronectin co-stimulation", [online], [searched on September 6, 2023], Internet <URL: https://catalog.takara-bio.co.jp/com/tech_info_detail.php?mode=3&masterid=M100004690&unitid=U100002954>

### Summary of Invention

### Technical Problem

Therefore, an object of the present invention is to provide a method for producing iPS cell-derived cytotoxic T cells having excellent cancer cytotoxic activity, a method for proliferating iPS cell-derived cytotoxic T cells, and the like.

### Solution to Problem

As a result of intensive studies to achieve the above object, the present inventors have studied scale-up of the activation step, and have found that by activating iPS-derived cytotoxic T cells using a container excellent in gas exchange, proliferation in an expansion culture step is equivalent to that when activation is performed using a T flask, and iPS-derived cytotoxic T cells excellent in cell proliferation and cancer cytotoxic activity after freeze-thawing are obtained after the expansion culture step.

The present invention has been completed by further conducting studies based on these findings, and provides the following method for producing cytotoxic T cells, method for proliferating cytotoxic T cells, cell population containing cytotoxic T cells, medicine, and the like.
[1] A method for producing activated cytotoxic T cells derived from iPS cells, the method comprising the following step (1): (1) activating unactivated cytotoxic T cells derived from iPS cells in a container excellent in gas exchange.
[2] The method according to [1], wherein the unactivated cytotoxic T cells contain a foreign gene encoding a receptor.
[3] The method according to [2], wherein the receptor is a chimeric antigen receptor.
[4] The method according to any one of [1] to [3], wherein the container is a bag.
[4a] The method according to any one of [1] to [3], wherein the container is a container in which a material excellent in gas permeability is used for a bottom surface.
[5] A method for proliferating activated cytotoxic T cells derived from iPS cells, the method comprising the following step (1A): (1A) activating unactivated cytotoxic T cells derived from iPS cells in a container excellent in gas exchange.
[6] The method according to [5], wherein the unactivated cytotoxic T cells contain a foreign gene encoding a receptor.
[7] The method according to [6], wherein the receptor is a chimeric antigen receptor.
[8] The method according to any one of [5] to [7], wherein the container is a bag.
[8a] The method according to any one of [5] to [7], wherein the container is a container in which a material excellent in gas permeability is used for a bottom surface.
[9] A cell population containing activated cytotoxic T cells obtained by the method according to any one of [1] to [8a].
[10] A medicine containing the cell population containing activated cytotoxic T cells according to [9].
[11] The medicine according to [10], which is an agent for preventing or treating a cancer.
[12] A method for preventing or treating a cancer, the method comprising administering the cell population containing activated cytotoxic T cells according to [9] to a subject in need thereof.
[13] The cell population containing activated cytotoxic T cells according to [9], for use in prevention or treatment of a cancer.
[14] Use of the cell population containing activated cytotoxic T cells according to [9] in the production of a medicine for use in prevention or treatment of a cancer.

### Advantageous Effects of Invention

According to the present invention, it is possible to produce iPS-derived cytotoxic T cells having excellent cancer cytotoxic activity.

### Brief Description of Drawings

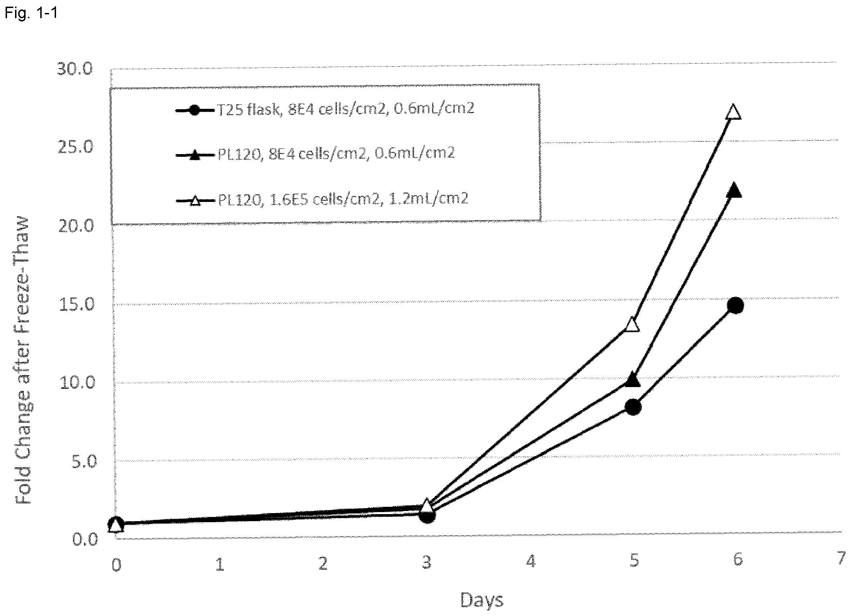

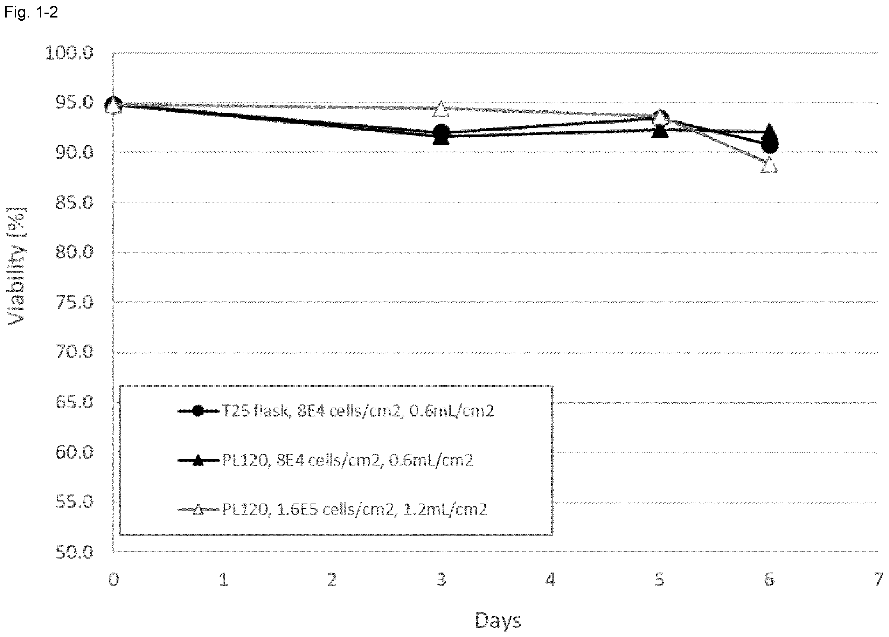

FIG. 1 is a graph showing the number of iPS cell-derived activated CAR-T cells (relative value when day 0 is taken as 1) (upper graph) and the viability (%) (lower graph) for 3 days of activation culture and for 3 days of expansion culture;

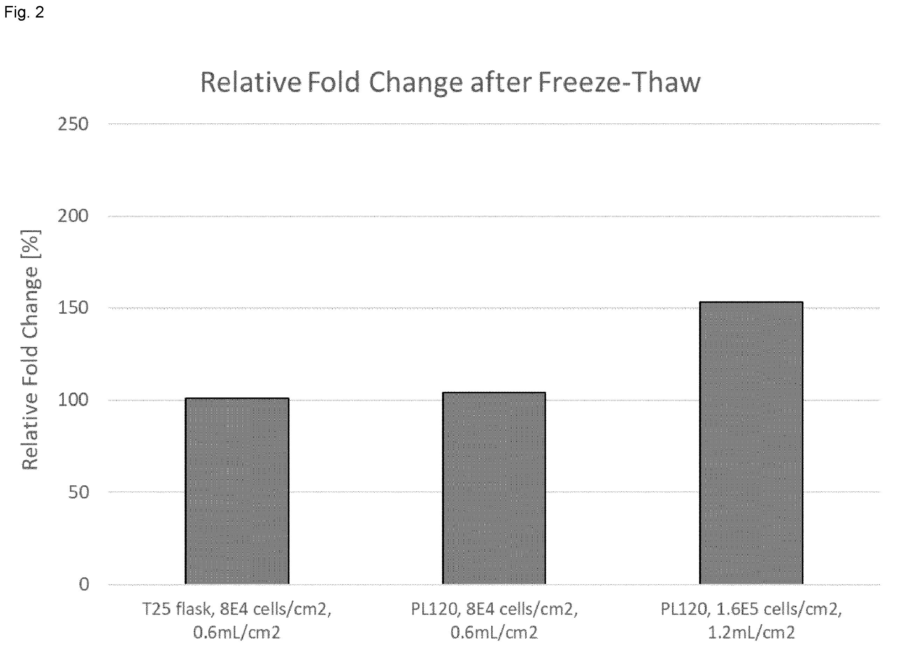

FIG. 2 is a graph showing results of a proliferation test of iPS cell-derived activated CAR-T cells from day 0 to day 7 of culture after freeze-thawing, in which vertical axis represents a relative value when the fold change of cell growth of iPS cell-derived activated CAR-T cells as a standard from day 0 to day 7 of culture is taken as 100%;

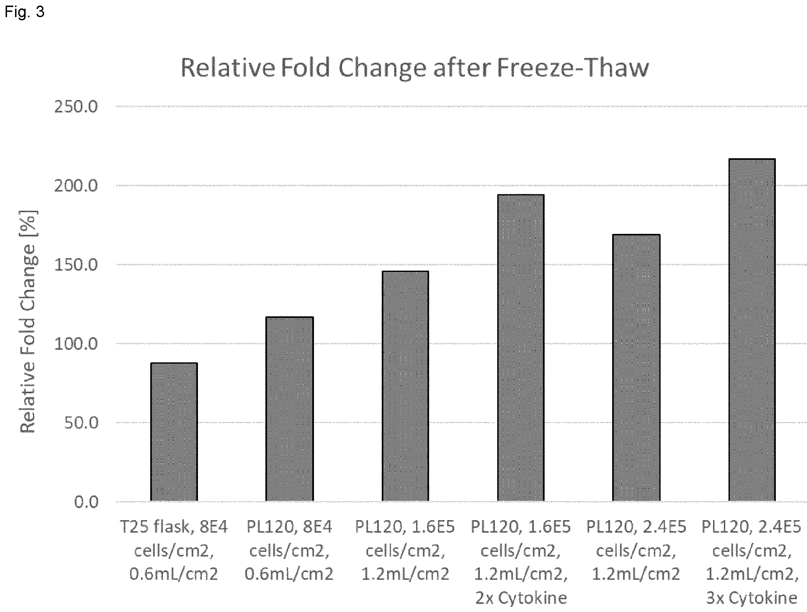

FIG. 3 is a graph showing results of a proliferation test of iPS cell-derived activated CAR-T cells from day 0 to day 7 of culture after freeze-thawing when the cytokine concentration in an activation step is increased, in which vertical axis represents a relative value when the fold change of cell growth of iPS cell-derived activated CAR-T cells as a standard from day 0 to day 7 of culture is taken as 100%;

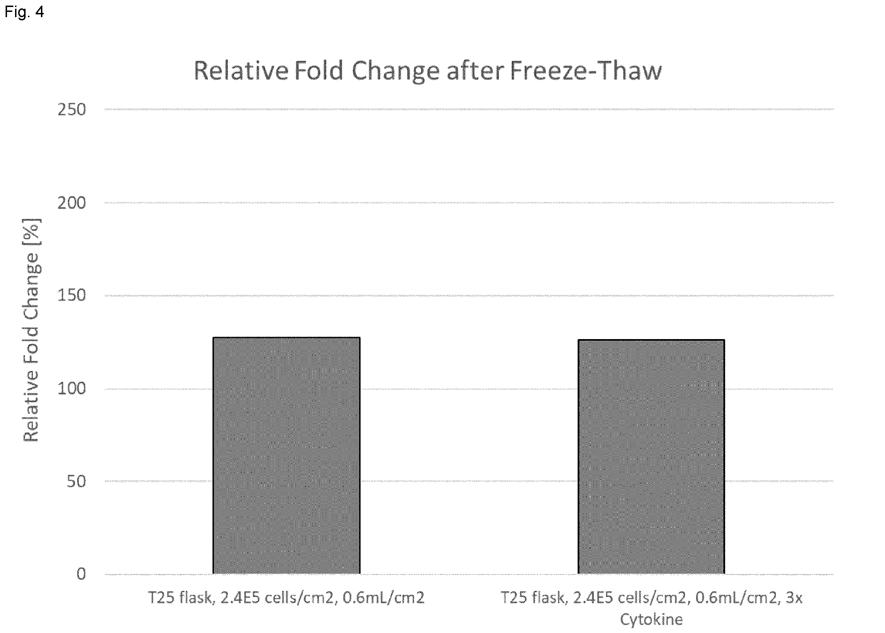

FIG. 4 is a graph showing results of a proliferation test of iPS cell-derived activated CAR-T cells from day 0 to day 7 of culture after freeze-thawing when the cytokine concentration in an activation step is increased, in which vertical axis represents a relative value when the fold change of cell growth of iPS cell-derived activated CAR-T cells as a standard from day 0 to day 7 of culture is taken as 100%;

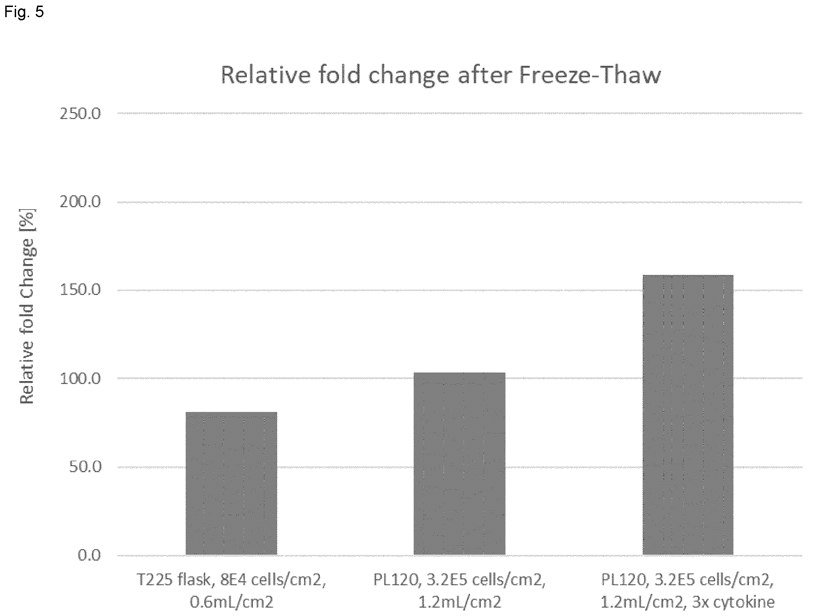

FIG. 5 is a graph showing results of a proliferation test of iPS cell-derived activated CAR-T cells from day 0 to day 7 of culture after freeze-thawing when the cytokine concentration in an activation step is increased, in which vertical axis represents a relative value when the fold change of cell growth of iPS cell-derived activated CAR-T cells as a standard from day 0 to day 7 of culture is taken as 100%; and

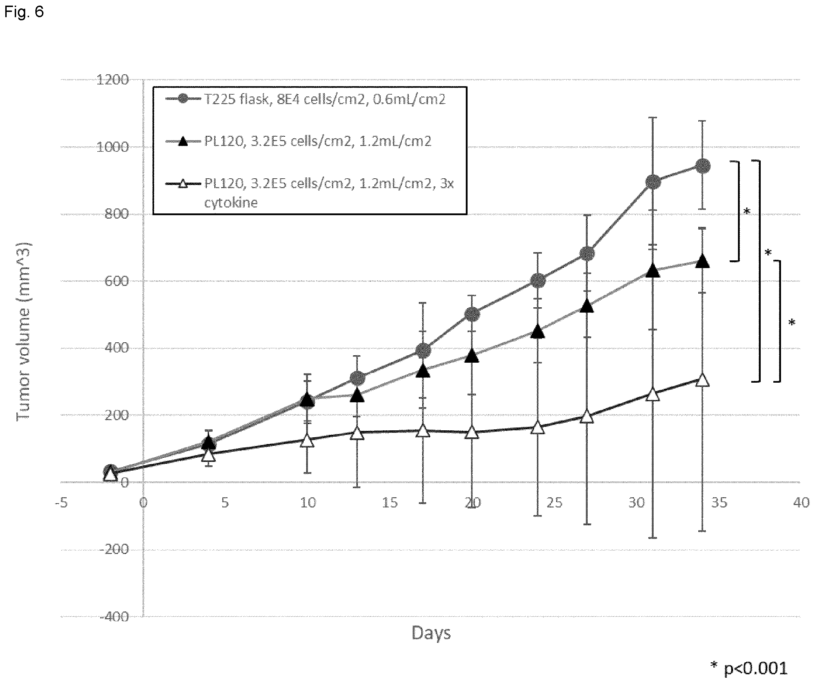

FIG. 6 is a graph showing the antitumor effect of iPS cell-derived activated CAR-T cell in vivo, in which the vertical axis represents the size (mm³) of a tumor of NSG mice carrying GSU cells, which are human gastric cancer-derived cell strains, and a value in the graph is mean value ± standard deviation, *p < 0.001, and significant difference test between respective groups was performed by two-way analysis of variance, N = 5;

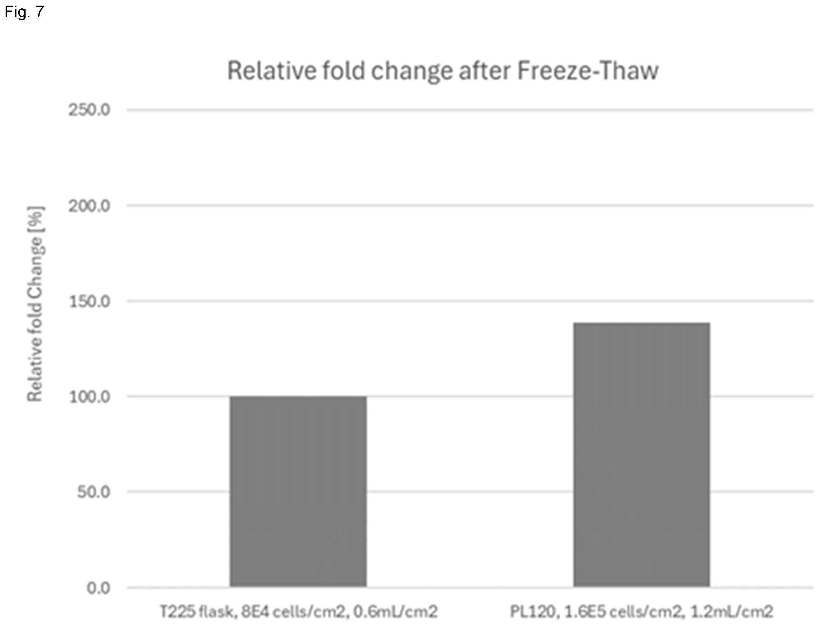

FIG. 7 is a graph showing results of a proliferation test of iPS cell-derived activated CAR-T cells from day 0 to day 6 of culture after freeze-thawing when iPS cell-derived activated CAR-T cells recognizing a different antigen were used, in which vertical axis represents a relative value when the fold change of cell growth of cells activated in a T225 flask from day 0 to day 6 of culture is taken as 100%;
FIG, 8 is a graph showing results of a proliferation test of iPS cell-derived activated CAR-T cells from day 0 to day 7 of culture after freeze-thawing when containers used in an activation step are different, in which vertical axis represents a relative value when the fold change of cell growth of iPS cell-derived activated CAR-T cells as a standard from from day 0 to day 7 of culture is taken as 100%; and

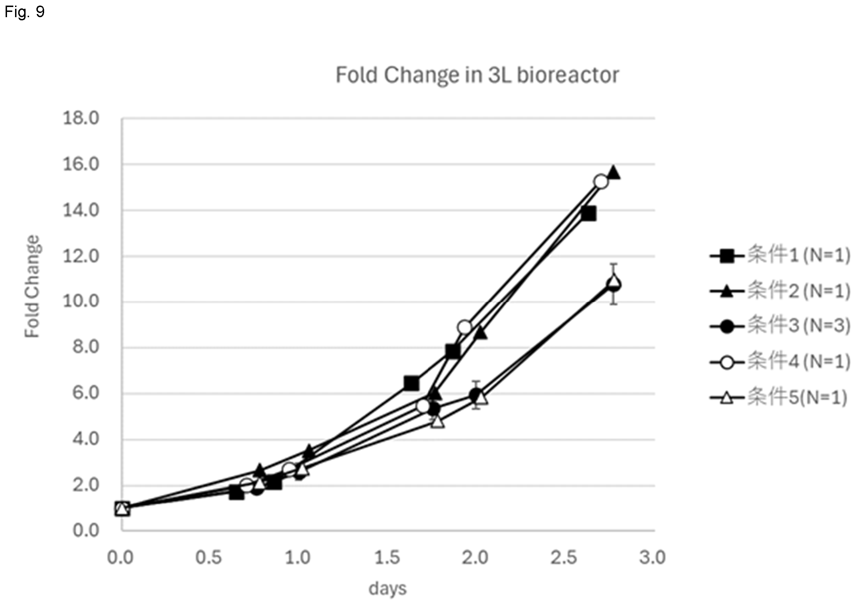

FIG. 9 is a graph showing the number of iPS cell-derived activated CAR-T cells (relative value when day 0 is taken as 1) for 3 days of expansion culture when a container used in an activation step is scaled up, condition 1: N = 1, condition 2: N = 1, condition 3: N = 3 (a value is a mean value ± standard deviation), condition 4: N= 1, and condition 5: N= 1.

### Description of Embodiments

Embodiments of the present invention are described in detail below.

The term "comprise(s)" or "comprising" means that although elements following these terms are included, the inclusion is not limited to the elements. Therefore, these terms suggest inclusion of elements following them, but do not suggest exclusion of any other elements. The term "consist(s) of" or "consisting of" means that any elements following these terms are included, and that the inclusion is limited to the elements. Therefore, the term "consist(s) of" or "consisting of" indicates that the listed elements are required or essential, and that there are substantially no other elements. The term "consist(s) essentially of" or "consisting essentially of" means that any elements following these terms are included, and that there is a limitation to other elements that do not affect the activity or action specified in the present disclosure for the above elements. Therefore, the term "consist(s) essentially of" or "consisting essentially of" indicates that the listed elements are required or essential, while other elements are optional, and may be or may not be present depending on whether they affect the activity or action of the listed elements.

In the present specification, the term "culture" refers to maintenance or/and proliferation of cells in an in-vitro environment. The term "culturing" refers to maintaining or/and proliferating cells outside tissue or outside the body (e.g., in a cell culture dish or flask).

In the present specification, the term "positive (+)" means that a protein or gene is expressed in detectable amounts by methods known in the art. In the case of a protein that is expressed intracellularly and does not appear on the cell surface (e.g., a transcription factor or a subunit thereof), a reporter protein is expressed together with the protein, and the reporter protein is detected, whereby the target protein can be detected. Gene detection can be performed by using nucleic acid amplification and/or nucleic acid detection methods, such as RT-PCR, microarray, biochip, and RNAseq.

In the present specification, the term "negative (-)" means that the expression level of a protein or gene is less than the lower limit of detection by all or any of the known methods described above, or the degree of expression thereof is low. The lower limit of detection of protein or gene expression may vary depending on the method. The degree of protein or gene expression (low expression or high expression) can be determined by comparison with the result of control cells measured under the same conditions.

Cell surface markers can be detected by immunological assays using antibodies specific for the cell surface markers, such as ELISA, immunostaining, and flow cytometry. In the present specification, the cell surface marker refers to a protein expressed on the cell surface that can be labeled (stained) with fluorescent substances and that facilitates the detection, condensation, isolation, or the like of cells expressing the cell surface marker. The cell surface marker refers to a gene that is expressed (positive marker) or not expressed (negative marker) specifically in a given cell type, and specifically a substance that is produced (positive marker) or not produced (negative marker) as mRNA by transcription of the gene in the genome or as a protein by translation of the mRNA.

In the present specification, the term "expression" is defined as the transcription and/or translation of a specific nucleotide sequence driven by an intracellular promoter.

In the present specification, "pluripotent stem cells" refer to embryonic stem cells (ES cells) and cells with similar pluripotency, i.e. cells with the potential to differentiate into various tissues (endoderm, mesoderm, and ectoderm) of a living body. Examples of cells with the same pluripotency as that of ES cells include induced pluripotent stem cells (also referred to as "iPS cells" in the present specification).

In the present specification, "hematopoietic progenitor cells" (HPC) are cells that have the ability to differentiate into blood cells but do not have the ability to self-renew as much as stem cells. Hematopoietic progenitor cells are mainly present in the bone marrow in a human living body. In the present specification, hematopoietic progenitor cells (HPC) can be positive to CD34 and negative to CD43 (CD34⁺/CD43⁺). Further, HPC can be positive to CD24, CD62L, CD90, CD143, CD263, Notch3, CD32, CD39, CD49a, CD164, CD317, CD200, CD218a, CD7, CD144, CD56, CD226, CD262, and CD325 and negative to CD49f, CD51, CD102, CD42b, CD61, CD62P, CD69, CD102, and CD156c, as described in WO2018/199186.

In the present specification, "hemogenic endothelial cells (HEC)" are cells that express CD34 but do not express CD43, CD184, and CD73 (CD34⁺/CD43⁻/CD184⁻/CD73⁻) (CD34⁺/CD43⁻/CD184⁻/CD73⁻ cells do not express CD7, and are thus also referred to as "CD34⁺/CD7⁻/CD43⁻/CD184⁻/CD73⁻ cells").

In the present specification, "cytotoxic T cells" are T cells that are positive for CD8 of a surface antigen and negative for CD4 (CD4⁻/CD8⁺, CD8 single positive (SP)) among T cells, and have cytotoxic activity. The cytotoxic T cells recognize an antigen peptide derived from a virus, a tumor, or the like presented together with a Class I major histocompatibility antigen (MHC Class I, HLA Class I) of antigen-presenting cells via a T cell receptor (TCR) present on the cell surface thereof, and specifically exert a cytotoxic activity against cells presenting the antigen peptide as a foreign substance. The cytotoxic activity can be confirmed by, for example, secretion or production of granzyme, perforin, or the like as an index.

In the present specification, "cell population" means two or more cells of the same type or different types. The "cell population" also means a mass of cells of the same type or different types.

In the present specification, "proliferation of cytotoxic T cells" or "proliferation of activated cytotoxic T cells" refers to an increase in the number (absolute number) of cytotoxic T cells in a cell population as compared to that before culture, and for example, means an increase of at least 1%, 5%, 10%, 20%, 30%, 40%, 50%, 60%, 70%, 80%, 90%, 100%, 120%, 150%, 200%, 300%, 400%, 500%, 600%, 700%, 800%, 900%, 1000%, 1200%, 1500%, 2000%, 2500%, or 3000% as compared to that before culture or to a control. In a particular embodiment of the present invention, cytotoxic T cells are produced so that the number (absolute number) of cytotoxic T cells in the cell population produced according to the present invention is an increase of at least 1%, 5%, 10%, 20%, 30%, 40%, 50%, 60%, 70%, 80%, 90%, 100%, 120%, 150%, 200%, 300%, 400%, 500%, 600%, 700%, 800%, 900%, 1000%, 1200%, 1500%, 2000%, 2500%, or 3000% as compared to that before culture.

The various cells used in the present invention are preferably cells certified by the Good Manufacturing Practice (GMP), in terms of therapeutic applications.

"Induced pluripotent stem cells" refer to cells obtained by reprogramming mammalian somatic cells or undifferentiated stem cells by introduction with specific factors (nuclear reprogramming factors). Currently, there are many different types of induced pluripotent stem cells, and usable examples include iPS cells established by Yamanaka et al. by introducing four factors, Oct3/4, Sox2, Klf4, and c-Myc, into mouse fibroblasts (Takahashi K, Yamanaka S., Cell, (2006) 126: 663-676), as well as human cell-derived iPS cells established by introducing the same four factors into human fibroblasts (Takahashi K, Yamanaka S., et al. Cell, (2007) 131: 861-872), Nanog-iPS cells established by introducing the above four factors, followed by screening using the expression of Nanog as an index (Okita, K., Ichisaka, T., and Yamanaka, S. (2007). Nature 448, 313-317), iPS cells produced by a method that does not include c-Myc (Nakagawa M, Yamanaka S., et al. Nature Biotechnology, (2008) 26, 101-106), and iPS cells established by introducing six factors by a virus-free method (Okita K et al. Nat. Methods 2011 May; 8(5): 409-12, Okita K et al. Stem Cells. 31(3): 458-66). Other usable examples include induced pluripotent stem cells produced by Thomson et al. and established by introducing four factors, OCT3/4, SOX2, NANOG, and LIN28 (Yu J., Thomson JA. et al., Science (2007) 318: 1917-1920), induced pluripotent stem cells produced by Daley et al. (Park IH., Daley GQ. et al., Nature (2007) 451: 141-146), induced pluripotent stem cells produced by Sakurada et al. (JP2008-307007A), and the like.

Other usable examples are any of the induced pluripotent stem cells known in the art and disclosed in all of the published articles (e.g., Shi Y., Ding S., et al., Cell Stem Cell, (2008) Vol. 3, Issue 5, 568-574; Kim JB., Scholer HR., et al., Nature, (2008) 454, 646-650; Huangfu D., Melton, DA., et al., Nature Biotechnology, (2008) 26, No. 7, 795-797), or patents (e.g., JP2008-307007A, JP2008-283972A, US2008/2336610, US2009/047263, WO2007/069666, WO2008/118220, WO2008/124133, WO2008/151058, WO2009/006930, WO2009/006997, and WO2009/007852).

As induced pluripotent stem cell strains, various iPS cell strains established by NIH, RIKEN, Kyoto University, etc. can be used. Examples of human iPS cell strains include HiPS-RIKEN-1A strain, HiPS-RIKEN-2A strain, HiPS-RIKEN-12A strain, and Nips-B2 strain (RIKEN); Ff-I01s04 strain, QHJI strain, RWMH strain, DRXT strain, RJWI strain, YZWJ strain, ILCL strain, GLKV strain, 253G1 strain, 201B7 strain, 409B2 strain, 454E2 strain, 606A1 strain, 610B1 strain, and 648A1 strain (Kyoto University); and the like.

The "nucleic acid" may be any molecule obtained by polymerizing a nucleotide and a molecule having the same function as the nucleotide. Examples include RNA that is a polymer of ribonucleotide, DNA that is a polymer of deoxyribonucleotide, a polymer that is a mixture of ribonucleotide and deoxyribonucleotide, and a nucleotide polymer containing a nucleotide analog. The nucleic acid may also be a nucleotide polymer containing a nucleic acid derivative. The nucleic acid may be a single-stranded nucleic acid or a double-stranded nucleic acid. Double-stranded nucleic acids include a double-stranded nucleic acid in which one strand hybridizes to the other strand under stringent conditions.

The nucleotide analog may be any molecule as long as it is a molecule obtained by modifying ribonucleotide, deoxyribonucleotide, RNA, or DNA, for improvement of nuclease resistance, stabilization, increase in affinity with complementary strand nucleic acids, enhancement of cell permeability, or visualization, compared with RNA or DNA. The nucleotide analog may be a naturally occurring molecule or a non-natural molecule. Examples include sugar-modified nucleotide analogs (e.g., 2'-O-methylribose-substituted nucleotide analog, 2'-O-propylribose-substituted nucleotide analog, 2'-methoxyethoxyribose-substituted nucleotide analog, 2'-O-methoxyethylribose-substituted nucleotide analog, 2'-O-[2-(guanidium)ethyl]ribose-substituted nucleotide analog, 2'-fluororibose-substituted nucleotide analog, bridged nucleic acid (BNA), locked nucleic acid (LNA), ethylene-bridged nucleic acid (ENA), peptide nucleic acid (PNA), oxy-peptide nucleic acid (OPNA), and peptide ribonucleic acid (PRNA)), phosphodiester bond-modified nucleotide analogs (e.g., phosphorothioate bond-substituted nucleotide analog and N3'-P5' phosphoramidate bond-substituted nucleotide analog), and the like.

The nucleic acid derivative may be any molecule as long as it is a molecule obtained by adding another chemical substance to a nucleic acid, for improvement of nuclease resistance, stabilization, increase in affinity with complementary strand nucleic acids, enhancement of cell permeability, or visualization, compared with the nucleic acid. Specific examples include 5'-polyamine-adduct derivatives, cholesterol-adduct derivatives, steroid-adduct derivatives, bile acid-adduct derivatives, vitamin-adduct derivatives, Cy5-adduct derivatives, Cy3-adduct derivatives, 6-FAM-adduct derivatives, biotin-adduct derivatives, and the like.

The method for producing activated cytotoxic T cells derived from iPS cells of the present invention (also referred to simply as "the production method of the present invention" in the present specification) characteristically includes the following step (1):
(1) activating unactivated cytotoxic T cells derived from iPS cells in a container excellent in gas exchange (also referred to as "activation step" in the present specification).

Unactivated cytotoxic T cells used in step (1) are induced to differentiate from iPS cells, and can be induced to differentiate into cytotoxic T cells according to a known method. Examples of such a method include inducing differentiation of iPS cells into hemogenic endothelial cells or hematopoietic progenitor cells, and then inducing differentiation of the hematopoietic stem cells or hematopoietic progenitor cells into cytotoxic T cells. Such differentiation of iPS cells into hemogenic endothelial cells or hematopoietic progenitor cells can be performed, for example, in accordance with the description of Nature Communications, 2021, 12, 430 and Molecular Therapy Methods & Clinical Development, 2020, 19, 250-260. Differentiation of hemogenic endothelial cells or hematopoietic progenitor cells into cytotoxic T cells can be performed in accordance with the description of Nature Communications, 2021, 12, 430 and WO2017/221975.

The unactivated cytotoxic T cells used in step (1) may be negative for CCR7 (CD197) and/or CD25, and the activated cytotoxic T cells activated in step (1) may be positive for CCR7 and/or CD25.

iPS cells and cells for producing iPS cells used in the present invention may be derived from humans, or may be derived from mammals other than humans (non-human mammals), and are preferably derived from humans. Examples of non-human mammals include mice, rats, hamsters, guinea pigs, rabbits, dogs, cats, pigs, cows, horses, sheep, and monkeys. Further, universalized iPS cells (having a specific HLA that does not cause immune rejection in a large number of patients, or having HLA knocked out) can also be used.

Examples of the substance used for activation of the unactivated cytotoxic T cells include a CD3 agonist, a CD28 agonist, and a CD30 agonist, examples of the CD3 agonist include an anti-CD3 antibody, examples of the CD28 agonist include an anti-CD28 antibody, and examples of the CD30 agonist include an anti-CD30 antibody. These can be used singly or in combination of two or more kinds thereof, and substances other than antibodies having similar effects can also be used. These antibodies may be monoclonal or polyclonal antibodies, but are preferably monoclonal antibodies. In the activation step, RetroNectin (trade name) (Recombinant Human Fibronectin Fragment, Takara Bio Inc.) may be used simultaneously with a CD3 agonist, a CD28 agonist, a CD30 agonist, or the like. The substance used for activation of the unactivated cytotoxic T cells and RetroNectin can also be used by binding them to a culture container.

The concentration of the substance used for activation of the unactivated cytotoxic T cells is not particularly limited as long as it is a concentration capable of stimulating a surface molecule of the cytotoxic T cells to transmit a signal within the cytotoxic T cells and inducing activation. For example, when an anti-CD3 antibody, an anti-CD28 antibody, and an anti-CD30 antibody are used, they can be used so that the final concentration of each antibody is 0.1 to 20 µg/mL.

The time of the activation step is not particularly limited as long as the activation of cytotoxic T cells can be induced. The time is, for example, 10 to 120 hours, preferably 15 to 100 hours, and more preferably 20 to 90 hours.

The activation step can be terminated by replacing the medium with a medium not containing the substance used for activation of the unactivated cytotoxic T cells or diluting the medium containing the substance used for activation of the unactivated cytotoxic T cells.

In the activation step of the present invention, except that a container excellent in gas exchange and iPS cell-derived unactivated cytotoxic T cells are used, other embodiments of culture, such as the culture method, medium, medium volume, and other culture conditions (temperature, atmosphere, cell density, etc.) are in accordance with known or common cytotoxic T cell activation methods, or can be suitably adjusted to suit the present invention based on the known or common cytotoxic T cell activation methods. The cell density at the time of seeding in the activation step can be, in terms of volume, for example, 1.0×10⁴ to 1.0×10⁷ cells/mL, and preferably 2.0×10⁴ to 6.0×10⁶ cells/mL; and can be, in terms of area, for example, 5.0×10³ to 5.0×10⁶ cells/cm², and preferably 2.5×10⁴ to 2.5×10⁶ cells/cm². The medium volume in the activation step can be, for example, 0.05 to 20 ml/cm², and can be preferably 0.1 to 10 ml/cm².

As the culture container in the activation step, a container excellent in gas exchange is used. The container excellent in gas exchange means a container having a structure capable of exchanging gas between the inside of the container or the medium and the outside of the container or the medium and having excellent gas exchange efficiency. Examples of the container excellent in gas exchange include a container having a structure capable of taking a gas outside the container into the container and discharging the gas inside the container to the outside of the container (for example, a bioreactor having a structure capable of taking a gas outside the container into the container and discharging the gas inside the container to the outside of the container), and a container in which a material excellent in gas permeability is used for a bottom surface (for example, a bag in which a material excellent in gas permeability (for example, fluoroethyl polymer) is used and a container in which a material having gas permeability (for example, silicone elastomer or a gas-permeable polystyrene) is used for a bottom surface). In the present specification, the gas is, for example, air, oxygen, carbon dioxide, nitrogen, or the like.

Examples of the container excellent in gas exchange include a container having oxygen (O₂) permeability of, for example, 1 to 10000 ml/24 hours (25°C), preferably 10 to 7000 ml/24 hours (25°C). In another embodiment, examples of the culture container used in the activation step include a container in which the permeability of carbon dioxide (CO₂) is, for example, 10 to 20000 ml/24 hours (25°C), preferably 50 to 15000 ml/24 hours (25°C), and the permeability of nitrogen (N₂) is, for example, 5 to 5000 ml/24 hours (25°C), preferably 100 to 2500 ml/24 hours (25°C).

The type of the container can be appropriately selected from plates, dishes, petri dishes, flasks, bags, bottles, tanks (culture tanks), bioreactors, and the like. Examples of the container excellent in gas exchange include a container in which a material excellent in gas permeability is used (e.g., a bag in which a material excellent in gas permeability (for example, fluoroethyl polymer) is used and a container in which a material having gas permeability (for example, silicone elastomer or a gas-permeable polystyrene) is used for a bottom surface), and a bioreactor capable of ventilation (e.g., a bioreactor equipped with a gas supply device). Examples of commercially available products of containers excellent in gas exchange include PL07-2G, PL30-2G, PL70-2G, PL120-2G, PL240-2G, PL325-2G, PL500P-2G, PL750-2G, PL1000P-2G, and PL2000P2-2G of OriGen Biomedical; CultiLife 215 Culture bag and GT-T610 (CultiLife Eva) Culture bag of Takara Bio Inc.; G-Rex (trade name) 10, G-Rex 10M, G-Rex 10M-CS, G-Rex 100, G-Rex 100M, G-Rex 100-CS, G-Rex 24 Well Plate, G-Rex 6 Well Plate, and G-Rex 6M Well Plate of Wilson Wolf; and other MACS (trade name) GMP Cell Culture Bags of Miltenyi Biotec, VueLife (trade name) "C" Series Bags, VueLife (trade name) "AC" Series Bags, and VueLife (trade name) "HP" Series Bags of Saint-Gobain S.A., NIPRO Cell Culture Bag of NIPRO CORPORATION, and HYPERFLASK (trade name) and HYPERSTACK (trade name) of Corning Incorporated.

In the activation step, a known or general medium used for culturing cytotoxic T cells can be used, and the medium can be appropriately supplemented with necessary components.

Examples of the medium in the activation step include AIM V, X-VIVO-15, NeuroBasal, EGM2, TeSR, BME, BGJb, CMRL 1066, Glasgow MEM, Improved MEM Zinc Option, IMDM, Medium 199, Eagle MEM, αMEM, DMEM, Ham, RPMI-1640, Fisher medium, and other various commercially available products for culturing T cells (e.g., CTS OpTmizer (trade name) T-Cell Expansion Basal Medium (Thermo Fisher Scientific), CTS OpTmizer (trade name) Pro Serum Free Medium (Thermo Fisher Scientific), CTS OpTmizer (trade name) Pro (Thermo Fisher Scientific), CTS OpTmizer (trade name) T-Cell Expansion Supplement (Thermo Fisher Scientific), and 4CELL (trade name) Nutri-T medium (Sartorius)). These media may be used singly or as a mixture of two or more.

The medium may be a serum-containing medium or a serum-free medium, or a xeno-free medium. From the viewpoint of preventing contamination with xenogeneic animal-derived components, the serum may be derived from the same animal as the cells to be cultured. The serum-free medium refers to a medium without unprocessed or unpurified serum, and thus can include a medium with purified blood-derived components or animal tissue-derived components (such as growth factors). The medium may or may not contain any serum replacements. Serum replacements can include materials suitably containing albumin (lipid-rich albumin, bovine albumin, albumin substitutes such as recombinant albumin or humanized albumin, plant starch, dextran, protein hydrolysates, and the like), transferrin (or other iron transporters), fatty acids, insulin, collagen precursors, trace elements, 2-mercaptoethanol, 3'-thioglycerol (α-monothioglycerol, MTG), or equivalents thereof. Commercially available materials such as knockout serum replacement (KSR), chemically-defined lipid concentrated (Thermo Fisher Scientific), and GlutaMAX (Thermo Fisher Scientific) can also be used.

The medium may contain one or two or more kinds selected from the group consisting of biotin; DL alpha tocopherol acetate; DL alpha-tocopherol; vitamins such as vitamins A (acetate); BSA (bovine serum albumin) or human albumin, fatty acid-free fraction V; catalase; human recombinant insulin; human transferrin; proteins such as superoxide dismutase; corticosterone; D-galactose; ethanolamine HCl; glutathione (reduced form); L-carnitine HCl; linoleic acid; linolenic acid; progesterone; putrescine 2HCl; sodium selenite; T3 (triiodo-L-thyronine); PSG (penicillin, streptomycin, and L-glutamine); and a caspase inhibitor. The medium may contain ascorbic acid or a derivative thereof (for example, ascorbic acid 2-phosphate: PAA) added from the outside. The medium may contain externally added one or two or more kinds selected from the group consisting of fatty acids or lipids, amino acids (such as non-essential amino acids), vitamins, growth factors, cytokines, antibiotics, antioxidants, 2-mercaptoethanol, pyruvic acid, buffers, and inorganic salts.

The medium may contain a cytokine added from the outside. Examples of the cytokine include FLT3 ligand (FLT3L), interleukin 7 (IL-7), stem cell factor (SCF), thrombopoietin (TPO), IL-2, IL-3, IL-4, IL-6, IL-12, IL-15, IL-18, IL-21, TNF-alpha, TGF-beta, interferon-gamma, interferon-lambda, TSLP, thymopentin, pleotrophin, and midkine. Any one of these cytokines may be used singly or two or more thereof may be used in combination. In one embodiment, IL-2, IL-7, IL-15, IL-18, and IL-21 are preferable as cytokines to be added to the medium, and it is more preferable to use these cytokines in combination. When these cytokines are used in combination, preferred concentrations thereof are as follows:
IL-7: 0.3 to 100 ng/mL (preferably 1.0 to 30 ng/mL, more preferably 3.0 to 20 ng/mL)
IL-15: 0.3 to 100 ng/mL (preferably 1.0 to 30 ng/mL, more preferably 3.0 to 20 ng/mL)
IL-2: 3 to 5000 IU/mL (preferably 10 to 3000 IU/mL, more preferably 30 to 1500 IU/mL)
IL-18: 2 to 1000 ng/ml (preferably 10 to 500 ng/mL, more preferably 30 to 300 ng/mL, further preferably 50 to 200 ng/mL, still more preferably 100 to 180 ng/mL)
IL-21: 1 to 1000 ng/ml (preferably 3 to 300 ng/mL, more preferably 10 to 150 ng/mL, further preferably 20 to 100 ng/mL, still more preferably 50 to 80 ng/mL)

In one embodiment of the present invention, unactivated cytotoxic T cells to be activated may be unactivated cytotoxic T cells containing a foreign gene encoding a receptor. The "foreign gene" is a gene to be introduced from the outside in order to express the desired protein in the cytotoxic T cells, and can be suitably selected depending on the use application of the cytotoxic T cells. The foreign gene may be one or more kinds. Examples of the receptor include a chimeric antigen receptor (CAR), a chimeric costimulatory receptor (CCR), a synthetic TCR and antigen receptor (STAR), TCR, and a chimeric TAC (T cell antigen coupler) receptor.

The foreign gene can be, for example, a gene for expressing a chimeric antigen receptor (CAR), and can further contain a gene for expressing a cytokine and/or a chemokine. As with general or known CARs, the CARs expressed by the cytotoxic T cells are basically configured such that peptides at sites of (i) an antigen recognition site that recognizes cell surface antigens of cancer cells (e.g., single-chain antibody), (ii) a transmembrane region, and (iii) a signal transduction region that induces the activation of T cells, are linked via a spacer, as needed. The foreign gene can also be, for example, a gene for expressing an exogenous T-cell receptor (TCR). The exogenous TCR means that it is exogenous to cytotoxic T cells into which a nucleic acid encoding the exogenous TCR is to be introduced. The amino acid sequence of the exogenous TCR may be identical to or different from that of the endogenous TCR of the T cells. One or two or more foreign genes may be introduced (e.g., CAR and exogenous TCR).

The means for introducing the foreign gene into the cells is not particularly limited, and various known or general means can be used. Typically, the foreign gene is introduced into the cells using an expression vector, and is expressed. The expression vector may be linear or cyclic, and may be a non-viral vector such as a plasmid, or a transposon vector. The cells into which the foreign gene is introduced are not particularly limited, and may be at any stage of differentiation, and examples thereof include iPS cells, cytotoxic T cells, hematopoietic progenitor cells, and hemogenic endothelial cells.

The means for introducing the expression vector into cells can be made appropriate according to the embodiment. For example, the expression vector can be introduced into cells by a known method, such as a virus infection method, a calcium phosphate method, a lipofection method, a microinjection method, or an electroporation method. The expression vector can be prepared in a form suitable for use in each method by known means, or using commercially available kits as appropriate (according to the instructions thereof).

The expression vector can be introduced into cells by a virus infection method. Examples of viral vectors include retroviral vectors, lentiviral vectors, adenoviral vectors, and adeno-associated viral vectors. When using these viral vectors, a vector containing the nucleic acid for expressing the receptor and a packaging vector (plasmid) of each virus may be transfected into host cells using a corresponding commercially available kit to produce a recombinant virus, and then cells may be infected with the obtained recombinant virus.

When a plurality of foreign genes are used, all of the foreign genes may be contained in one expression vector, all of the foreign genes may be separately contained in different expression vectors, or some of the plurality of foreign genes may be contained in one expression vector, and the others may be separately contained in different expression vectors. When the plurality of foreign genes are contained in one expression vector, the order in which those foreign genes are arranged from the upstream side to the downstream side is not particularly limited.

The foreign gene can be composed of a nucleic acid (polynucleotide) having a base sequence encoding the amino acid sequence of a desired receptor. Those skilled in the art can design and produce an expression vector capable of expressing a desired receptor in the cells. The nucleic acid contained in the expression vector may be produced by chemical DNA synthesis reaction or may be produced (cloned) as cDNA.

The expression vector may contain the sequence of a promoter, a terminator, an enhancer, a start codon, a stop codon, a polyadenylation signal, a nuclear localization signal (NLS), a multicloning site (MCS), or the like, as needed, in addition to the nucleic acid encoding the desired receptor. The expression vector may further contain a nucleic acid (base sequence) encoding "functional genes," such as reporter genes (e.g., genes encoding various color fluorescent proteins), drug selection genes (e.g., kanamycin resistance gene, ampicillin resistance gene, and puromycin resistance gene), and suicide genes (e.g., genes encoding diphtheria A toxin, herpes simplex virus thymidine kinase (HSV-TK), carboxypeptidase G2 (CPG2), carboxylesterase (CA), cytosine deaminase (CD), cytochrome P450 (cyt-450), deoxycytidine kinase (dCK), nitroreductase (NR), purine nucleoside phosphorylase (PNP), thymidine phosphorylase (TP), varicella zoster virus thymidine kinase (VZV-TK), xanthine-guanine phosphoribosyltransferase (XGPRT), inducible caspase 9, etc.).

The production method of the present invention can further include the following step (2) and/or step (3):
(2) expanding the cytotoxic T cells activated by the activation step by culture, and then cryopreserving the cytotoxic T cell expanded by culture (referred to as "cryopreservation step" in the present specification); and
(3) thawing and culturing the activated cytotoxic T cells cryopreserved in the cryopreservation step (referred to as "culture step" in the present specification).

The cryopreservation step is a step of expanding the activated cytotoxic T cells obtained in the activation step, and then cryopreserving the cytotoxic T cell. The culture period of the expansion culture is not particularly limited as long as it is a period sufficient to obtain the desired number of cells, and is, for example, 1 to 20 days, preferably 2 to 7 days.

The culture container, medium, added components, and other culture conditions in expansion culture of the cryopreservation step can be the same as those described with respect to the activation step, and for example, can be appropriately selected from those listed. However, the culture container, medium, added components, and the like in expansion culture of the cryopreservation step are not necessarily the same as those in the activation step, and can be appropriately changed. In particular, it is not essential to use a container excellent in gas exchange, and a culture container in which gas exchange is not possible or gas exchange efficiency is low may be used. The substance used for activation of the unactivated cytotoxic T cells may not be contained in the medium.

Cryopreservation of cytotoxic T cells expanded by culture can employ known means. The cytotoxic T cells can be appropriately stored at an optimum temperature, for example, about -10°C or less, about -30°C or less, about -80°C or less, or the like, and can be cryopreserved in a liquid nitrogen tank, a deep freezer, or the like. The temperature during cryopreservation may fluctuate somewhat during storage, and in particular, it is preferable to keep it within the temperature range described above from freezing to being useful for use. The period of cryopreservation of the cytotoxic T cells is not particularly limited, and storage for several months to several decades is also possible. As a preservation solution for preserving cytotoxic T cells, an aqueous solution, such as a buffer solution, an isotonic solution, or a medium, which contains a cryoprotectant generally widely used for cryopreservation of animal cells, can be used. Examples of such a cryoprotectant include dimethyl sulfoxide, ethylene glycol (EG), propylene glycol (PG), 1,2-propanediol (1,2-PD), 1,3-propanediol (1,3-PD), butylene glycol (BG), isoprene glycol (IPG), dipropylene glycol (DPG), and glycerin. The content of the cryoprotectant is not particularly limited, and may be, for example, 2 to 10 vol%. Examples of the container for cryopreserving cytotoxic T cells include a tube, a vial, a well plate, and a cell freezing container.

The culture step is a step of thawing and culturing the activated cytotoxic T cells cryopreserved in the cryopreservation step. The culture period here is not particularly limited as long as it is a period sufficient to obtain the desired number of cells, and is, for example, 1 to 20 days, preferably 2 to 10 days.

The method for thawing the cryopreserved activated cytotoxic T cells is not particularly limited, and known means can be adopted. For example, known means and conditions such as a method of rapidly melting in a warm bath at 37°C and a method of gently melting at room temperature can be adopted.

The culture container, medium, added components, and other culture conditions in culture of the culture step can be the same as those described with respect to the activation step, and for example, can be appropriately selected from those listed. However, the culture container, medium, added components, and the like in culture of the culture step are not necessarily the same as those in the activation step, and can be appropriately changed. In particular, it is not essential to use a container excellent in gas exchange, and a culture container in which gas exchange is not possible or gas exchange efficiency is low may be used. The substance used for activation of the unactivated cytotoxic T cells may not be contained in the medium.

The activated cytotoxic T cells in the culture step have higher cell proliferation (after cryopreservation) compared to the case of activating iPS cell-derived unactivated cytotoxic T cells in a container (for example, T flask) in which gas exchange is not possible or gas exchange efficiency is low (control group). The activated cytotoxic T cells obtained after the culture step have higher cancer cytotoxic activity (after cryopreservation) compared to the case of activating iPS cell-derived unactivated cytotoxic T cells in a container in which gas exchange is not possible or gas exchange efficiency is low (control group).

The method for proliferating activated cytotoxic T cells derived from iPS cells of the present invention (also referred to simply as "the proliferation method of the present invention" in the present specification) characteristically includes the following step (1A):
(1A) activating unactivated cytotoxic T cells derived from iPS cells in a container excellent in gas exchange.

Step (1A) can be performed in the same manner as in step (1). The description of the production method of the present invention applies to the proliferation method of the present invention.

The production method and the proliferation method of the present invention can also be applied to iPS cell-derived natural killer (NK) cells in addition to the iPS cell-derived cytotoxic T cells. That is, it is possible to provide a method for producing iPS cell-derived activated NK cells or a method for proliferating iPS cell-derived activated NK cells, which uses iPS cell-derived unactivated NK cells. Unactivated NK cells are induced to differentiate from iPS cells, and can be induced to differentiate into NK cells according to a known method. Examples of such a method include inducing differentiation of iPS cells into a cell population containing CD34⁺HPC, then inducing differentiation into a cell population containing CD4⁻cells, and further inducing differentiation into a cell population enriched in NK cells. Such differentiation of iPS cells into NK cells can be performed in accordance with the description of WO 2022/264033. The NK cells are CD56⁺ cells, and may be further CD56⁺/CD16⁺ cells or CD56⁺/CD3⁻ cells. Examples of the substance used for activation of the unactivated NK cells include IL-2, IL-7, IL-15, IL-21, IL-18, substances that stimulate molecules with immunoreceptor tyrosine-based activation motifs (ITAMs) such as NKp46 (ligands, antibodies, ligand-fusion Fc proteins, etc.), and substances that stimulate molecules such as 4-1BB, OX40, CD28, ICOS, and CD30 (ligands, antibodies, ligand-fusion Fc proteins, etc.).

The use application of the cell population containing cytotoxic T cells obtained by the production method and the proliferation method of the present invention is not particularly limited, and the cell population can be used for any desired purpose, and for example, can be used for producing a pharmaceutical (cell preparation). The proportion (number of cells) of activated cytotoxic T cells contained in the cell population containing activated cytotoxic T cells obtained by the production method and the proliferation method of the present invention is, for example, 5% or more, preferably 15% or more, more preferably 25% or more, further preferably 35% or more, and still more preferably 45% or more, and the upper limit is, for example, 100% or less.

The medicine (cell preparation) contains cytotoxic T cells, and may further contain other components as needed. Those skilled in the art can suitably prepare such a medicine using cytotoxic T cells in consideration of the use application (disease to be treated, subject to be administered, or the like) and dosage form.

The medicine can be, for example, a medicine (anticancer agent) to treat and prevent a cancer corresponding to the cell surface antigen of cancer cells (cancer-specific antigen) targeted by the CAR expressed by cytotoxic T cells. Therefore, the type of cancer targeted by the medicine is not particularly limited as long as cancer cells expressing the antigen targeted by the CAR are contained in the cancer tissue, and a certain level of therapeutic and prophylactic effects are observed by the CAR-T cells. Examples of the cancer that can be targeted by the medicine include cancers such as adenocarcinoma, squamous cell carcinoma, adenosquamous carcinoma, undifferentiated cancer, large-cell cancer, small-cell cancer, skin cancer (e.g., melanoma and Merkel cell cancer), breast cancer, prostate cancer, bladder cancer, vaginal cancer, neck cancer, head and neck cancer, uterine cancer, cervical cancer, liver cancer, kidney cancer, pancreatic cancer, spleen cancer, lung cancer, non-small-cell lung cancer, tracheal cancer, bronchial cancer, colon cancer, rectal cancer, small-intestine cancer, colorectal cancer, stomach cancer, esophageal cancer, gallbladder cancer, testicular cancer, ovarian cancer, fallopian tube cancer, and nasopharyngeal cancer; cancers of bone tissue, cartilage tissue, adipose tissue, muscle tissue, vascular tissue, and hematopoietic tissue; sarcomas such as chondrosarcoma, Ewing's sarcoma, rhabdomyosarcoma, malignant hemangioendothelioma, osteosarcoma, and soft tissue sarcoma; blastomas such as hepatoblastoma, medulloblastoma, nephroblastoma, neuroblastoma, pancreatoblastoma, pleuropulmonary blastoma, and retinoblastoma; embryonic cell tumors; lymphoma; leukemia, acute myeloid leukemia, and multiple myeloma.

Examples of components that can be contained in the medicine other than the cytotoxic T cells include pharmaceutically acceptable additives, more specifically, saline, buffered saline, cell culture media, dextrose, water for injection, glycerol, ethanol, stabilizers, solubilizers, surfactants, buffers, preservatives, isotonic agents, fillers, and lubricants.

The medicine can be used by being administered to a subject in need of treatment and prevention of cancer (such as cancer patients and cancer-bearing animals) by the same method as for known cytotoxic T cells (for example, CAR-T cells). Examples of the administration method include intratumor, intravenous, intraarterial, intramuscular, subcutaneous, and intraperitoneal injections.

The amount of cytotoxic T cells contained in the medicine can be appropriately adjusted depending on use application, dosage form, intended therapeutic and prophylactic effects, and the like, for example, in consideration of the type, location, and severity of cancer, the age, body weight, and condition of the subject to be treated, and the like. For example, the medicine can be formulated so that the CAR-T cells are administered in an amount of normally 1×10⁴ to 1×10¹⁰, preferably 1×10⁵ to 5×10⁹, and more preferably 5×10⁶ to 2×10⁹, in a single dose.

The administration interval of the medicine is not particularly limited, and can be appropriately adjusted in consideration of the amount of the T cells of the present invention to be administered at one time, and the medicine can be independently administered, for example, 4 times, 3 times, twice, or once a day, every other day, every 3 days, every 4 days, every 5 days, every 6 days, once a week, every 8 days, every 9 days, every 10 days, twice a week, once a month, or twice a month.

When the medicine is used for cancer treatment and prevention, it can be used in combination with a known anticancer agent. Examples of the anticancer agent include alkylating drugs such as cyclophosphamide, bendamustine, ifosfamide, and dacarbazine; antimetabolites such as pentostatin, fludarabine, cladribine, methotrexate, 5-fluorouracil, 6-mercaptopurine, and enocitabin; molecular targeted drugs such as rituximab, cetuximab, and trastuzumab; kinase inhibitors such as imatinib, gefitinib, erlotinib, afatinib, dasatinib, sunitinib, and trametinib; proteasome inhibitors such as bortezomib; calcineurin inhibitors such as cyclosporine and tacrolimus; antitumor antibiotics such as idarubicin, doxorubicin, and mitomycin C; plant alkaloids such as irinotecan and etoposide; platinum preparations such as cisplatin, oxaliplatin, and carboplatin; hormone therapeutic drugs such as tamoxifen and bicalutamide; and immunoregulatory drugs such as interferon, nivolumab, and pembrolizumab. When the target recognition site of CAR is an antibody that recognizes the constant region of the antibody that specifically binds to a cell surface antigen of cancer cells, the cytotoxic T cells can be used as a medicine for treating and preventing a cancer that corresponds to the cell surface antigen. In this case, a medicine containing cytotoxic T cells can be used in combination with a medicine containing an antibody that specifically binds to the cell surface antigen.

Singular terms shall include pluralities and plural terms shall include the singular when being used in the present specification and claims unless otherwise required by context. Therefore, articles of singular forms (for example, "a", "an", "the", and the like in English) should be understood to include the concept of plural forms unless otherwise specified.

### Examples

Hereinafter, the present invention will be described in detail with reference to Examples, but the present invention is not limited thereto at all. Reagents and materials to be used are commercially available unless otherwise specified, or can be prepared by known literature or the like. Those having similar effects and actions can be substituted.

### (List of Abbreviations)

BMP-4: bone morphogenetic protein-4
bFGF: basic fibroblast growth factor
VEGF: vascular endothelial growth factor
SCF: stem cell factor
TPO: thrombopoietin
FLT3L: Fms-related tyrosine kinase 3 ligand
Fc-DLL4: Recombinant Human DLL4 Fc Chimera Protein
DLL4: Delta-like protein 4
IL7: Interleukin-7
SDF1α: Stromal cell-derived factor 1α
αMEM: alpha Modified Eagle Minimum Essential Medium
TCR: T-cell receptor

### Example 1 Study on Influence of Container Used in Activation Step on Proliferation Efficiency of iPS Cell-Derived Activated CAR-T Cells After Freeze-Thawing

### (1) Preparation of iPS Cell-Derived CAR-T Cells

A TCR gene was transduced into an iPS cell QHJI01S04 strain donated from Kyoto University iPS Cell Research Institute (CiRA) using a lentiviral vector. iPS cells into which the TCR gene has been transduced were differentiated into hematopoietic progenitor cells according to the method described in Nature Communication 2021; 12: 430. Specifically, iPS cells were cultured for 4 days in the presence of CHIR99021, SB431542, BMP-4, bFGF, and VEGF to induce differentiation into mesoderm. Further, the cells were differentiated into hematopoietic progenitor cells using SCF, TPO, and FLT3L which are hematopoietic cytokines. Differentiation of the obtained hematopoietic progenitor cells into cytotoxic T cells (CTL; Cytotoxic T Lymphocyte) was performed according to WO2017/221975 and Nature Communication 2021; 12: 430. Specifically, among the obtained hematopoietic progenitor cells, CD34 positive cells purified using magnetic beads (Myltenyi Biotec) were cultured for 3 weeks in an αMEM medium containing SCF, TPO, FLT3L, IL7, SDF1α, and SB203580 on a plate obtained by immobilizing Fc-DLL4 and RetroNectin (Recombinant Human Fibronectin Fragment, Takara Bio Inc.). A CAR gene recognizing mesothelin (using the base sequence described in WO2023/009700) was transduced into obtained TCR-positive CTL using a retroviral vector. Hereinafter, cells obtained by cryopreserving the cells with liquid nitrogen were used as iPS cell-derived CAR-T cells.

### (2) Recovery Culture of iPS Cell-Derived CAR-T Cells

The iPS cell-derived CAR-T cells were suspended in a medium obtained by adding an the additives shown in the "Recovery culture medium" column of Table 1 to an IMDM medium containing 15% Fetal Bovine Serum (FBS), at 500,000 cells/mL, seeded on G-Rex 10M or G-Rex 6M (Wilson Wolf), and cultured for 3 days at 5% CO₂/37°C.

**[Table 1]**

| Additive to medium | Manufacturer | Final concentration | Recovery culture medium | Activation culture medium | Expansion culture medium |
|---|---|---|---|---|---|
| L-Glutamine (200 mM) | Thermo Fisher Scientific | 2 mM | + | + | + |
| Streptomycin Sulfate | Meiji Seika Pharma | 100 µg/mL | + | + | + |
| ITS (100x) (Insulin-Transferrin-Selenium Supplements) | Thermo Fisher Scientific | 1x | + | + | + |
| Ascorbic Acid 2-Phosphate | Sigma-Aldrich | 50 µg/mL | + | + | + |
| IL-7 | PeproTech | 10 ng/mL | + | + | + |
| IL-15 | PeproTech | 10 ng/mL | + | + | + |
| IL-2 | PeproTech | 1280 IU/mL | + | + | + |
| IL-18 | MBL | 50 ng/mL | | + | |
| IL-21 | PeproTech | 20 ng/mL | | + | + |
| Caspase Inhibitor Z-VAD-FMK | R&D Systems | 10 µM | | + | |
| Human CD30 antibody | R&D Systems | 300 ng/mL | | + | + |

### (3) Reagent and Antibody

As an anti-CD3 agonist antibody, Anti-CD3 mAb GMP grade, Anti-CD3 monoclonal antibody (Clone: OKT3) purchased from Takara Bio Inc. was used.

### (4) Immobilization of Anti-CD3 Agonist Antibody to Culture Plate

The anti-CD3 agonist antibody (OKT3, final concentration: 3 µg/mL) dissolved in PBS at the required concentration was added to a T25 flask and PermaLife Cell Culture Bag PL120 (OriGen Biomedical), followed by standing still overnight at 4°C. After washing with PBS, the resultant was subjected to a test.

### (5) Activation Culture of iPS Cell-Derived CAR-T Cells

The iPS cell-derived CAR-T cells after recovery culture were seeded on a T25 flask and PL120 on which an anti-CD3 agonist antibody (OKT3) was immobilized in a medium obtained by adding the additives shown in the "Activation culture medium" column of Table 1 to an IMDM medium containing 15% FBS, with the medium volume and the cell amount in Table 2. After seeding, each was cultured for 3 days at 5% CO₂/37°C. iPS cell-derived CAR-T cells as a standard used in a proliferation test after freeze-thawing were produced by performing activation culture using T225 flask and performing other culture steps using the same method. The same applies to cells described as iPS cell-derived CAR-T cells as a standard in the following Examples.

**[Table 2]**

| Activation culture container | Number of cells at time of activation culture | Medium volume at time of activation culture |
|---|---|---|
| T25 flask | 80,000 cells/cm² | 0.6 mL/cm² |
| PL120 | 80,000 cells/cm² | 0.6 mL/cm² |
| PL120 | 160,000 cells/cm² | 1.2 mL/cm² |

### (6) Expansion Culture of iPS Cell-Derived Activated CAR-T Cells

The iPS cell-derived activated CAR-T cells after activation culture were suspended in a medium obtained by adding the additives shown in the "Expansion culture medium" column of Table 1 to an IMDM medium containing 15% FBS, at 40,000 cells/mL, and cultured using G-Rex 6M (Wilson Wolf) for 3 days at 5% CO₂/37°C. On day 2 of culture, some cells were recovered from G-Rex 6M, the number of cells was measured, and the medium was replaced with an expansion culture medium. On day 3 of culture, some cells were recovered from G-Rex (trade name) 6M, and the number of cells was measured. All cells after culture were recovered, centrifuged, and then cryopreserved in a cryopreservation solution containing 5% DMSO.

The number of cells and the viability of iPS cell-derived activated CAR-T cells were measured on days 0, 2, and 3 of expansion culture in the above (6). The fold change of cell growth and the viability of cells for 3 days of activation culture and for 3 days of expansion culture are shown in FIG. 1. According to this study, it was found that iPS cell-derived activated CAR-T cells activated and cultured in PL120 proliferate in expansion culture.

### (7) Proliferation Test After Freeze-Thawing of iPS Cell-Derived Activated CAR-T Cells

Cryopreserved iPS cell-derived activated CAR-T cells were thawed, and the cells were suspended in an IMDM medium (containing 15% FBS, 2 mM L-Glutamine, 100 µg/mL Streptomycin, 1xITS, 50 µg/mL Ascorbic Acid 2-phosphate) at 125,000 cells/mL, and cultured using a G-Rex 24-well plate (Wilson Wolf) at 5% CO₂/37°C. Thereafter, on day 1 and day 3 of culture, some cells were recovered from the G-Rex 24-well plate, the number of cells was measured, and on day 3 of culture, the cells were suspended again in an IMDM medium (containing 15% FBS, 2 mM L-Glutamine, 100 µg/mL Streptomycin, 1xITS, 50 µg/mL Ascorbic Acid 2-phosphate) at 125,000 cells/mL, and cultured using a G-Rex 24-well plate at 5% CO₂/37°C. On day 7 of culture, some cells were recovered from the G-Rex 24-well plate, the number of cells was measured, and the fold change of cell growth from day 0 to day 7 of culture was calculated. iPS cell-derived CAR-T cells as a standard were simultaneously tested, and relative values when the fold change of cell growth from day 0 to day 7 of culture was taken as 100% were calculated and compared for each condition.

The results of the proliferation test from day 0 to day 7 of culture after freeze-thawing are shown in FIG. 2. It was found that the iPS cell-derived activated CAR-T cell activated and cultured in PL120 are excellent in proliferation capability after freeze-thawing.

### Example 2 Study on Influence of Cytokine Concentration in Activation Step on Proliferation Efficiency of iPS Cell-Derived Activated CAR-T Cells After Freeze-Thawing

### (1) Recovery Culture of iPS Cell-Derived CAR-T Cells

The recovery culture of iPS cell-derived CAR-T cells was performed by the method described in (2) of Example 1. As a medium in the recovery culture, a medium obtained by adding the additives shown in the "Recovery culture medium" column of Table 3 to an IMDM medium containing 15% Fetal Bovine Serum (FBS) was used.

**[Table 3-1]**

| Additive to medium | Manufacturer | Final concentration | Recovery culture medium | Activation culture medium | Activation culture medium (2× cytokine) | Activation culture medium (3× cytokine) | Expansion culture medium |
|---|---|---|---|---|---|---|---|
| L-Glutamine (200 mM) | Thermo Fisher Scientific | 2 mM | + | + | + | + | + |
| Streptomycin Sulfate | Meiji Seika Pharma | 100 µg/mL | + | + | + | + | + |
| ITS (100x) (Insulin-Transferrin-Selenium Supplements) | Thermo Fisher Scientific | 1x | + | + | + | + | + |
| Ascorbic Acid 2-Phosphate | Sigma-Aldrich | 50 µg/mL | + | + | + | + | + |
| IL-7 | PeproTech | 10 ng/mL | + | + | + | + | + |
| IL-15 | PeproTech | 10 ng/mL | + | + | + | + | + |
| IL-2 | PeproTech | 1280 IU/mL | + | + | + | + | + |
| IL-18 | MBL | 50 ng/mL | | + | | | |
| | | 100 ng/mL | | | + | | |
| | | 150 ng/mL | | | | + | |
| IL-21 | PeproTech | 20 ng/mL | | + | | | + |
| | | 40 ng/mL | | | + | | |
| | | 60 ng/mL | | | | + | |

**[Table 3-2]**

| | | | | | | | |
|---|---|---|---|---|---|---|---|
| Caspase Inhibitor Z-VAD-FMK | R&D Systems | 10 µM | | + | + | + | |
| Human CD30 antibody | R&D Systems | 300 ng/mL | | + | | | + |
| | | 600 ng/mL | | | + | | |
| | | 900 ng/mL | | | | + | |

### (2) Activation Culture of iPS Cell-Derived CAR-T Cells

The iPS cell-derived CAR-T cells after recovery culture were seeded on a T25 flask and PL120 on which an anti-CD3 agonist antibody (OKT3) was immobilized in a medium obtained by adding the additives shown in the "Activation culture medium", "Activation culture medium (2×cytokine)", and "Activation culture medium (3×cytokine)" columns of Table 3 to an IMDM medium containing 15% FBS, with the medium volumes and the cell amounts in Tables 4 and 5. After seeding, each was cultured for 3 days at 5% CO₂/37°C. The T25 flask and PL120 on which an anti-CD3 agonist antibody (OKT3) was immobilized were prepared by the methods described in (3) and (4) of Example 1.

**[Table 4]**

| Activation culture container | Number of cells at time of activation culture | Medium at time of activation culture | Medium volume at time of activation culture |
|---|---|---|---|
| T25 flask | 80,000 cells/cm² | Medium for activation | 0.6 mL/cm² |
| PL120 | 80,000 cells/cm² | Medium for activation | 0.6 mL/cm² |
| PL120 | 160,000 cells/cm² | Medium for activation | 1.2 mL/cm² |
| PL120 | 160,000 cells/cm² | Medium for activation (2× cytokine) | 1.2 mL/cm² |
| PL120 | 240,000 cells/cm² | Medium for activation | 1.2 mL/cm² |
| PL120 | 240,000 cells/cm² | Medium for activation (3× cytokine) | 1.2 mL/cm² |

**[Table 5]**

| Activation culture container | Number of cells at time of activation culture | Medium at time of activation culture | Medium volume at time of activation culture |
|---|---|---|---|
| T25 flask | 240,000 cells/cm² | Medium for activation | 0.6 mL/cm² |
| T25 flask | 240,000 cells/cm² | Medium for activation (3× cytokine) | 0.6 mL/cm² |

### (3) Expansion Culture of iPS Cell-Derived Activated CAR-T Cells

The expansion culture of iPS cell-derived activated CAR-T cells was performed by the method described in (6) of Example 1, and the cells on day 3 of culture were recovered and cryopreserved.

### (4) Proliferation Test After Freeze-Thawing of iPS Cell-Derived Activated CAR-T Cells

The proliferation of the iPS cell-derived activated CAR-T cells after freeze-thawing was measured by the method described in (7) of Example 1.

The results of the proliferation test from day 0 to day 7 of culture after freeze-thawing are shown in FIGS. 3 and 4. In the iPS cell-derived activated CAR-T cells activated and cultured in PL120, it was found that the proliferation capability after freeze-thawing was enhanced by enhancing the cytokine concentration in the activation culture (FIG. 3). On the other hand, in the iPS cell-derived activated CAR-T cells activated and cultured in the T25 flask, proliferation after freeze-thawing was not enhanced even when the cytokine concentration in the activation culture was enhanced (FIG. 4).

### Example 3 Study on Influence of Container Used in Activation Step on Antitumor Effect of iPS Cell-Derived Activated CAR-T Cells After Freeze-Thawing

### (1) Recovery Culture of iPS Cell-Derived CAR-T Cells

The recovery culture of iPS cell-derived CAR-T cells was performed using G-Rex 100M (Wilson Wolf) by the method described in (2) of Example 1. As a medium in the recovery culture, a medium obtained by adding the additives shown in the "Recovery culture medium" column of Table 6 to an IMDM medium containing 15% Fetal Bovine Serum (FBS) was used.

**[Table 6]**

| Additive to medium | Manufacturer | Final concentration | Recovery culture medium | Activation culture medium | Activation culture medium (3× cytokine) | Expansion culture medium |
|---|---|---|---|---|---|---|
| L-Glutamine (200 mM) | Thermo Fisher Scientific | 2 mM | + | + | + | + |
| Streptomycin Sulfate | Meiji Seika Pharma | 100 µg/mL | + | + | + | + |
| ITS (100x) (Insulin-Transferrin-Selenium Supplements) | Thermo Fisher Scientific | 1x | + | + | + | + |
| Ascorbic Acid 2-Phosphate | Sigma-Aldrich | 50 µg/mL | + | + | + | + |
| IL-7 | PeproTech | 10 ng/mL | + | + | + | + |
| IL-15 | PeproTech | 10 ng/mL | + | + | + | + |
| IL-2 | PeproTech | 1280 IU/mL | + | + | + | + |
| IL-18 | MBL | 50 ng/mL | | + | | |
| | | 150 ng/mL | | | + | |
| IL-21 | PeproTech | 20 ng/mL | | + | | + |
| | | 60 ng/mL | | | + | |
| Caspase Inhibitor Z-VAD-FMK | R&D Systems | 10 µM | | + | + | |
| Human CD30 antibody | R&D Systems | 300 ng/mL | | + | | + |
| | | 900 ng/mL | | | + | |

### (2) Activation Culture of iPS Cell-Derived CAR-T Cells

The iPS cell-derived CAR-T cells after recovery culture were seeded on a T225 flask and PL120 on which an anti-CD3 agonist antibody (OKT3) was immobilized in a medium obtained by adding the additives shown in the "Activation culture medium" and "Activation culture medium (3×cytokine)" columns of Table 6 to an IMDM medium containing 15% FBS, with the number of cells and the medium volume in Table 7. After seeding, the cells were cultured for 3 days at 5% CO₂/37°C.

The T225 flask and PL120 on which an anti-CD3 agonist antibody (OKT3) was immobilized were prepared by the methods described in (3) and (4) of Example 1.

**[Table 7]**

| Activation culture container | Number of cells at time of activation culture | Medium at time of activation culture | Medium volume at time of activation culture |
|---|---|---|---|
| T225 flask | 80,000 cells/cm² | Medium for activation | 0.6 mL/cm² |
| PL120 | 320,000 cells/cm² | Medium for activation | 1.2 mL/cm² |
| PL120 | 320,000 cells/cm² | Medium for activation (3× cytokine) | 1.2 mL/cm² |

### (3) Expansion Culture of iPS Cell-Derived Activated CAR-T Cells

The iPS cell-derived activated CAR-T cells after activation culture were suspended in a medium obtained by adding the additives shown in the "Expansion culture medium" column of Table 6 to an IMDM medium containing 15% FBS, at 40,000 cells/mL, and cultured using G-Rex 100M (Wilson Wolf) for 3 days at 5% CO₂/37°C. The medium was replaced with an expansion culture medium on day 2 of culture. On day 3 of culture, the cells were recovered from G-Rex 100M, centrifuged, and then cryopreserved in a cryopreservation solution containing 5% DMSO.

### (4) Proliferation Test After Freeze-Thawing of iPS Cell-Derived Activated CAR-T Cells

Cryopreserved iPS cell-derived activated CAR-T cells were thawed, and the cells were suspended in an IMDM medium (containing 15% FBS, 2 mM L-Glutamine, 100 µg/mL Streptomycin, 1xITS, 50 µg/mL Ascorbic Acid 2-phosphate) at 125,000 cells/mL, and cultured using a G-Rex 24-well plate (Wilson Wolf) at 5% CO₂/37°C. Thereafter, on day 1 and day 3 of culture, some cells were recovered from the G-Rex 24-well plate, the number of cells was measured, and on day 3 of culture, the cells were suspended again in an IMDM medium (containing 15% FBS, 2 mM L-Glutamine, 100 µg/mL Streptomycin, 1xITS, 50 µg/mL Ascorbic Acid 2-phosphate) at 125,000 cells/mL, and cultured using a G-Rex 24-well plate at 5% CO₂/37°C. On day 7 of culture, some cells were recovered from the G-Rex 24-well plate, and the number of cells was measured.

After freeze-thawing, the fold change of cell growth on day 7 of culture is shown in FIG. 5. It was found that the iPS cell-derived activated CAR-T cells activated and cultured in PL120 were superior in proliferation capability after freeze-thawing to the iPS cell-derived activated CAR-T cells activated and cultured in the T225 flask.

### (5) In Vivo Antitumor Effect of iPS Cell-Derived Activated CAR-T Cells After Freeze-Thawing in Human Tumor-Bearing Mouse Model

NSG mice were subcutaneously carried with 2,000,000 cells/mouse of GSU cells which are human gastric cancer-derived cell strains. After 7 days, the iPS cell-derived activated CAR-T cells cryopreserved after expansion culture were intravenously administered at 1,000,000 cells/mouse. The tumor size was measured with a 3D scanner every week until week 6 after administration. The test was performed with N = 5, and the mean value and standard deviation of the tumor size were calculated. A significant difference test between respective groups was performed by two-way analysis of variance.

The antitumor effect of iPS cell-derived activated CAR-T cell in vivo is shown in FIG. 6. The antitumor effect of the iPS cell-derived activated CAR-T cells activated and cultured in PL120 was significantly improved as compared to the iPS cell-derived activated CAR-T cells activated and cultured in the T225 flask. The antitumor effect was further improved by increasing the cytokine concentration at the time of activation culture.

### Example 4 Study on Influence of CAR-T Cells into Which CAR Gene Recognizing Different Antigen, CD19 Was Introduced on Proliferation Efficiency of iPS Cell-Derived Activated CAR-T Cells After Freeze-Thawing

### (1) Recovery Culture of iPS Cell-Derived CAR-T Cells

iPS cell-derived CAR-T cells into which a CAR gene recognizing CD19 has been introduced were produced by the same method as the method described in (1) of Example 1, and the cryopreserved cells were used below as iPS cell-derived CAR-T cells. The iPS cell-derived CAR-T cells were seeded on a T225 flask (Thermo Fisher Scientific) at 666,666 cells/mL in a medium obtained by adding the additives shown in the "Recovery culture medium" column of Table 1 to an IMDM medium containing 15% Fetal Bovine Serum (FBS), and cultured for 4 days at 5% CO₂/37°C. Similarly, the iPS cell-derived CAR-T cells were seeded on G-Rex 6M (Wilson Wolf) at 500,000 cells/mL in a medium obtained by adding all the additives shown in the "Recovery culture medium" column of Table 1, except for Streptomycin Sulfate, to an IMDM medium containing 15% Fetal Bovine Serum (FBS), and cultured for 4 days at 5% CO₂/37°C.

### (2) Activation Culture of iPS Cell-Derived CAR-T Cells

The iPS cell-derived CAR-T cells after recovery culture were seeded on a T225 flask and PL120 on which an anti-CD3 agonist antibody (OKT3) was immobilized, with the number of cells and the medium volume in Table 8. A medium obtained by adding the additives shown in the "Activation culture medium" column of Table 1 to an IMDM medium containing 15% FBS is used for the T225 flask, and a medium obtained by adding all the additives shown in the "Activation culture medium" column of Table 1, except for Streptomycin Sulfate, to an IMDM medium containing 15% FBS is used for the PL120, After seeding, each was cultured for 3 days at 5% CO₂/37°C. The T225 flask and PL120 on which an anti-CD3 agonist antibody (OKT3) was immobilized were prepared by the methods described in (3) and (4) of Example 1.

**[Table 8]**

| Recovery culture container | Activation culture container | Number of cells at time of activation culture | Medium volume at time of activation culture |
|---|---|---|---|
| T225 flask | T225 flask | 80,000 cells/cm² | 0.6 mL/cm² |
| G-Rex 6M | PL120 | 160,000 cells/cm² | 1.2 mL/cm² |

### (3) Expansion Culture of iPS Cell-Derived Activated CAR-T Cells

The expansion culture of iPS cell-derived activated CAR-T cells was performed by the method described in (6) of Example 1, and the cells on day 3 of culture were recovered and cryopreserved. The expansion culture of cells activated in PL120 was performed using a medium obtained by adding all the additives shown in the "Expansion culture medium" column of Table 1, except for Streptomycin Sulfate, to an IMDM medium containing 15% FBS.

### (4) Proliferation Test After Freeze-Thawing of iPS Cell-Derived Activated CAR-T Cells

Cryopreserved iPS cell-derived activated CAR-T cells were thawed, and the cells were suspended in an IMDM medium (containing 15% FBS, 2 mM L-Glutamine, 100 µg/mL Streptomycin, 1xITS, 50 µg/mL Ascorbic Acid 2-phosphate) at 125,000 cells/mL, and cultured using a G-Rex 24-well plate (Wilson Wolf) at 5% CO₂/37°C. Thereafter, on day 3 of culture, some cells were recovered from the G-Rex 24-well plate, the number of cells was measured, and on day 3 of culture, the cells were suspended again in an IMDM medium (containing 15% FBS, 2 mM L-Glutamine, 100 µg/mL Streptomycin, 1xITS, 50 µg/mL Ascorbic Acid 2-phosphate) at 125,000 cells/mL, and cultured using a G-Rex 24-well plate at 5% CO₂/37°C. On day 6 of culture, some cells were recovered from the G-Rex 24-well plate, the number of cells was measured, and the fold change of cell growth from day 0 to day 6 of culture was calculated. Relative values when the fold change of cell growth from day 0 to day 6 of culture of cells activated in T225 flask was taken as 100% were calculated and compared.

The results of the proliferation test from day 0 to day 6 of culture after freeze-thawing are shown in FIG. 7. Also in the iPS cell-derived activated CAR-T cells recognizing different antigens, it was found that the proliferation capability after freeze-thawing was enhanced in the case of activation in PL120 (FIG. 7).

### Example 5 Study on Influence of Container Used in Activation Step on Proliferation Efficiency of iPS Cell-Derived Activated CAR-T Cells After Freeze-Thawing

### (1) Recovery Culture of iPS Cell-Derived CAR-T Cells

The recovery culture of iPS cell-derived CAR-T cells was performed by the method described in (2) of Example 1. As a medium in the recovery culture, a medium obtained by adding the additives shown in the "Recovery culture medium" column of Table 3 to an IMDM medium containing 15% Fetal Bovine Serum (FBS) was used.

### (2) Activation Culture of iPS Cell-Derived CAR-T Cells

The iPS cell-derived CAR-T cells after recovery culture were seeded on a T25 flask, PL120, and G-Rex 6M on which an anti-CD3 agonist antibody (OKT3) was immobilized in a medium obtained by adding the additives shown in the "Activation culture medium" column of Table 1 to an IMDM medium containing 15% FBS, with the number of cells and the medium volume in Table 9. After seeding, each was cultured for 3 days at 5% CO₂/37°C. The T25 flask, PL120, and G-Rex 6M on which an anti-CD3 agonist antibody (OKT3) was immobilized were prepared by the same methods as the methods described in (3) and (4) of Example 1.

**[Table 9]**

| Activation culture container | Number of cells at time of activation culture | Medium volume at time of activation culture |
|---|---|---|
| T25 flask | 80,000 cells/cm² | 0.6 mL/cm² |
| PL120 | 80,000 cells/cm² | 0.6 mL/cm² |
| PL120 | 280,000 cells/cm² | 1.2 mL/cm² |
| G-REX 6M | 80,000 cells/cm² | 0.6 mL/cm² |
| G-REX 6M | 280,000 cells/cm² | 1.2 mL/cm² |
| G-REX 6M | 280,000 cells/cm² | 2.4 mL/cm² |
| G-REX 6M | 560,000 cells/cm² | 2.4 mL/cm² |
| G-REX 6M | 560,000 cells/cm² | 3.6 mL/cm² |
| G-REX 6M | 840,000 cells/cm² | 3.6 mL/cm² |

### (3) Expansion Culture of iPS Cell-Derived Activated CAR-T Cells

The expansion culture of iPS cell-derived activated CAR-T cells was performed by the method described in (6) of Example 1, and the cells on day 3 of culture were recovered and cryopreserved.

### (4) Proliferation Test After Freeze-Thawing of iPS Cell-Derived Activated CAR-T Cells

The proliferation of the iPS cell-derived activated CAR-T cells after freeze-thawing was evaluated by the method described in (7) of Example 1. In this Example, relative values when the fold change of cell growth from day 0 to day 7 of culture of cells activated in T25 flask (800,000 cells/cm², 0.6 mL/cm²) was taken as 100% were calculated and compared.

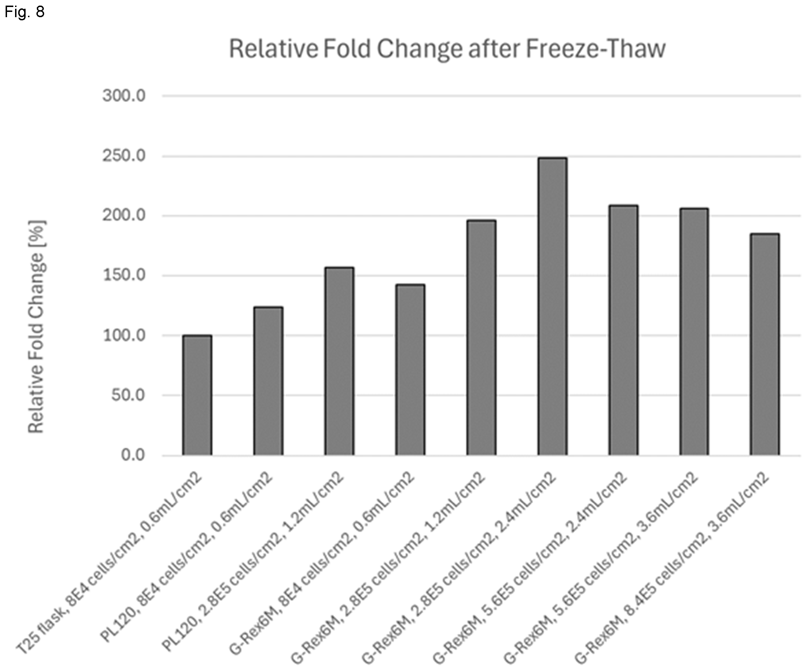

The results of the proliferation test from day 0 to day 7 of culture after freeze-thawing are shown in FIG. 8. The proliferation capability after freeze-thawing was enhanced also in the case of performing activation in G-REX 6M without being limited to PL120 (FIG. 8).

### Example 6 Study on Influence of Scaling-Up of Container Used in Activation Step on Proliferation Efficiency of iPS Cell-Derived Activated CAR-T Cells After Freeze-Thawing

### (1) Recovery Culture of iPS Cell-Derived CAR-T Cells

The recovery culture of iPS cell-derived CAR-T cells was performed by the method described in (2) of Example 1, except that G-Rex 100M was used as a culture container and the recovery culture medium and the recovery culture period were changed. As a medium in the recovery culture, a medium obtained by adding the additives shown in the "Recovery culture medium 1", "Recovery culture medium 2", and "Recovery culture medium 3" columns of Table 10 to an IMDM medium containing 15% Fetal Bovine Serum (FBS) was used. The recovery culture period was set to the conditions shown in Table 11.

**[Table 10-1]**

| Additive to medium | Manufacturer | Final concentration | Recovery culture medium 1 | Recovery culture medium 2 | Recovery culture medium 3 | Activation culture medium 1 | Activation culture medium 2 | Activation culture medium 3 |
|---|---|---|---|---|---|---|---|---|
| L-Glutamine (200 mM) | Thermo Fisher Scientific | 2 mM | + | + | + | + | + | + |
| Streptomycin Sulfate | Meiji Seika Pharma | 100 µg/mL | + | | | + | | |
| ITS (100x) (Insulin-Transferrin-Selenium Supplements) | Thermo Fisher Scientific | 1x | + | + | + | + | + | + |
| Ascorbic Acid 2-Phosphate | Sigma-Aldrich | 50 µg/mL | + | + | + | + | + | + |
| IL-7 | PeproTech | 10 ng/mL | + | + | + | + | + | + |
| IL-15 | PeproTech | 10 ng/mL | + | + | | + | + | |
| | | 100 ng/mL | | | + | | | + |

**[Table 10-2]**

| | | | | | | | | |
|---|---|---|---|---|---|---|---|---|
| IL-2 | PeproTech | 1280 IU/mL | + | + | + | + | + | + |
| IL-18 | MBL | 50 ng/mL | | | | + | + | + |
| IL-21 | Peprotech | 20 ng/mL | | | | + | + | + |
| Caspase Inhibitor Z-VAD-FMK | R&D Systems | 10 µM | | | | + | + | + |
| Human CD30 antibody | R&D Systems | 300 ng/mL | | | | + | + | + |

**[Table 11]**

| Condition | Recovery culture medium | Recovery culture period |
|---|---|---|
| Condition 1-3 | Recovery culture medium 1 | For 3 days |
| Condition 4 | Recovery culture medium 2 | For 3 days |
| Condition 5 | Recovery culture medium 3 | For 4 days |

### (2) Activation Culture of iPS Cell-Derived CAR-T Cells

The iPS cell-derived CAR-T cells after recovery culture were seeded on PL2000 and PL1000 on which an anti-CD3 agonist antibody (OKT3) was immobilized in a medium obtained by adding the additives shown in the of "Activation culture medium 1", "Activation culture medium2", and "Activation culture medium 3" columns of Table 10 to an IMDM medium containing 15% FBS, with the medium volume and the cell amount in Table 12. After seeding, each was cultured for 3 days at 5% CO₂/37°C. The PL2000 and PL1000 on which an anti-CD3 agonist antibody (OKT3) was immobilized were prepared by the same methods as the methods described in (3) and (4) of Example 1.

**[Table 12]**

| Condition | Activation culture medium | Activation culture container | Number of cells at time of activation culture | Medium volume at time of activation culture |
|---|---|---|---|---|
| Condition 1 | Activation culture medium 1 | PL2000 | 80,000 cells/cm² | 0.6 mL/cm² |
| Condition 2 | Activation culture medium 1 | PL2000 | 160,000 cells/cm² | 1.2 mL/cm² |
| Condition 3 | Activation culture medium 1 | PL2000 | 320,000 cells/cm² | 1.2 mL/cm² |
| Condition 4 | Activation culture medium 2 | PL2000 | 320,000 cells/cm² | 1.2 mL/cm² |
| Condition 5 | Activation culture medium 3 | PL1000 | 280,000 cells/cm² | 1.2 mL/cm² |

### (3) Expansion Culture of iPS Cell-Derived Activated CAR-T Cells

The iPS cell-derived activated CAR-T cells after activation culture were suspended in a 3 L bioreactor at 1,800,000 cells/mL, and cultured at 5% CO₂/37°C. During culture, "Expansion culture medium 1", "Expansion culture medium 2", and , "Expansion culture medium 3" of Table 13 were used, the medium was appropriately replaced as shown in Table 14, and culture for 3 days was performed while maintaining dissolved oxygen and pH. The cells after culture were recovered, centrifuged, and then cryopreserved in a cryopreservation solution containing 5% DMSO. The medium composition of MB301 in Table 13 is shown in Table 15.

**[Table 13-1]**

| Additive to medium | Manufacturer | Final concentration | Expansion culture medium 1 | Expansion culture medium 2 | Expansion culture medium 3 |
|---|---|---|---|---|---|
| IMDM | Thermo Fisher Scientific | - | + | + | |
| BM301 | AJINOMOTO KOHJIN BIO CO., LTD. | - | | | + |
| FBS | Selbome | 15% | + | + | |
| L-Glutamine | Thermo Fisher Scientific or Ajinomoto Co., Inc. | 2 mM | + | + | + |
| Streptomycin Sulfate | Meiji Seika Pharma | 100 µg/mL | + | | |
| ITS (100x) (Insulin-Transferrin-Selenium Supplements) | Thermo Fisher Scientific | 1x | + | + | |
| ITS-X (100x) (Insulin-Transferrin-Selenium-Ethanolamine Supplements) | Thermo Fisher Scientific | 1x | | | + |
| Ascorbic Acid 2-Phosphate | Sigma-Aldrich | 50 µg/mL | + | + | + |
| IL-7 | PeproTech | 10 ng/mL | + | + | + |
| IL-15 | PeproTech | 10 ng/mL | + | + | + |
| IL-2 | PeproTech | 1280 IU/mL | + | + | + |

**[Table 13-2]**

| | | | | | |
|---|---|---|---|---|---|
| IL-21 | Peprotech | 20 ng/mL | + | + | + |
| Human CD30 antibody | R&D Systems | 300 ng/mL | + | + | + |
| Boric acid | Sigma-Aldrich | 18.5 µg/mL | | | + |
| Taurine | Sigma-Aldrich | 375 µg/mL | | | + |
| L-Carnosine | Hamari Chemicals, Ltd. | 4.5 mg/mL | | | + |
| lysophosphatidylcholine (LPC) (derived from soybean) | Sigma-Aldrich | 50 µg/mL | | | + |
| Human serum albumine | CSL Behring | 2 g/L | | | + |

**[Table 14]**

| Condition | Expansion culture medium (day 0 to day 2 of culture) | Expansion culture medium (day 2 to day 3 of culture) |
|---|---|---|
| Conditions 1 to 3 | Recovery culture medium 1 | Activation culture medium 1 |
| Condition 4 | Recovery culture medium 2 | Activation culture medium 2 |
| Condition 5 | Recovery culture medium 3 | Activation culture medium 3 |

**[Table 15]**

| Component name | Concentration (mg/L) |
|---|---|
| Glycine | 30 |
| L-Alanine | 25 |
| L-Arginine hydrochloride | 84 |
| L-Asparagine-H20 | 28 |
| L-Aspartic acid | 30 |
| L-Cystine 2HCI | 91.4 |
| L-Glutamic Acid | 75 |
| L-Histidine hydrochloride-H20 | 42 |
| L-Isoleucine | 105 |
| L-Leucine | 105 |
| L-Lysine hydrochloride | 146 |
| L-Methionine | 30 |
| L-Phenylalanine | 66 |
| L-Proline | 40 |
| L-Serine | 42 |
| L-Threonine | 95 |
| L-Tryptophan | 16 |
| L-Tyrosine 2Na 2H2O | 121 |
| L-Valine | 94 |
| Biotin | 0.013 |
| Choline chloride | 8.98 |
| D-Calcium pantothenate | 4 |
| Folic Acid | 4 |
| Niacinamide | 4 |
| Pyridoxine hydrochloride | 2.013 |
| Riboflavin | 0.4 |
| Thiamine hydrochloride | 4 |
| Vitamin B12 | 0.68 |
| i-Inositol | 12.6 |
| Calcium Chloride dihydrate (CaCl2 2H20) | 219 |
| Cupric sulfate (CuSO4-5H2O) | 0.0013 |
| Ferric Nitrate (Fe(NO3)3"9H2O) | 0.05 |
| Ferric sulfate (FeSO4-7H2O) | 0.417 |
| Magnesium Sulfate (MgSO4) (anhyd.) | 97.67 |
| Potassium Chloride (KCI) | 330 |
| Sodium Bicarbonate (NaHCO3) | 3024 |
| Sodium Chloride (NaCl) | 4350 |
| Sodium Phosphate monobasic (NaH2PO4-H2O) | 125 |
| Zinc sulfate (ZnSO4-7H2O) | 0.864 |
| Sodium Selenite | 0.005 |
| D-Glucose (Dextrose) | 4500 |
| HEPES | 5958 |
| Ethanolamine | 1.9 |
| Hypoxanthine Na | 2.39 |
| Linoleic Acid | 0.042 |
| Lipoic Acid | 0.105 |
| Putrescine 2HCl | 0.081 |
| Sodium Pyruvate | 110 |
| Thymidine | 0.365 |
| L-Glutathione reduced | 1 |
| Ammonium Metavanadate | 0.0003 |
| Manganous Chloride tetrahydrate (MnCl2 4H2O) | 0.00005 |

The proliferation rate of the iPS cell-derived activated CAR-T cells for 3 days of expansion culture is shown in FIG. 9. It was confirmed that a high proliferation rate was exhibited even when iPS cell-derived activated CAR-T cells activated using PL2000 and PL1000, which were scaled-up, were seeded on a 3 L bioreactor and expanded by culture (FIG. 9).

This application is based on patent application No. 2023-185541 filed on October 30, 2023 in Japan, the contents of which are encompassed in full herein.

## Claims

1. A method for producing activated cytotoxic T cells derived from iPS cells, the method comprising the following step (1):
(1) activating unactivated cytotoxic T cells derived from iPS cells in a container excellent in gas exchange.

2. The method according to claim 1, wherein the unactivated cytotoxic T cells contain a foreign gene encoding a receptor.

3. The method according to claim 2, wherein the receptor is a chimeric antigen receptor.

4. The method according to claim 1, wherein the container is a bag.

5. A method for proliferating activated cytotoxic T cells derived from iPS cells, the method comprising the following step (1A):
(1A) activating unactivated cytotoxic T cells derived from iPS cells in a container excellent in gas exchange.

6. The method according to claim 5, wherein the unactivated cytotoxic T cells contain a foreign gene encoding a receptor.

7. The method according to claim 6, wherein the receptor is a chimeric antigen receptor.

8. The method according to claim 5, wherein the container is a bag.

9. A cell population comprising activated cytotoxic T cells obtained by the method according to claim 1 or 5.

10. A medicine comprising the cell population containing activated cytotoxic T cells according to claim 9.

11. The medicine according to claim 10, which is an agent for preventing or treating a cancer.

12. A method for preventing or treating a cancer, the method comprising administering the cell population containing activated cytotoxic T cells according to claim 9 to a subject in need thereof.

13. The cell population containing activated cytotoxic T cells according to claim 9, for use in prevention or treatment of a cancer.

14. Use of the cell population containing activated cytotoxic T cells according to claim 9 in the production of a medicine for use in prevention or treatment of a cancer.
